(19) Europäisches Patentamt
European Patent Office
Office européen des brevets

(11) **EP 3 558 147 B1**

(12) **EUROPEAN PATENT SPECIFICATION**

(45) Date of publication and mention
of the grant of the patent:
**07.05.2025 Bulletin 2025/19**

(21) Application number: **17884041.9**

(22) Date of filing: **19.12.2017**

(51) International Patent Classification (IPC):
*A61B 17/94* (2006.01)   *A61B 1/313* (2006.01)
*G02B 23/26* (2006.01)   *A61B 1/005* (2006.01)
*A61B 1/05* (2006.01)   *A61B 1/07* (2006.01)
*G01B 11/25* (2006.01)   *G02B 23/24* (2006.01)
*A61B 1/00* (2006.01)   *A61B 1/06* (2006.01)

(52) Cooperative Patent Classification (CPC):
**G02B 23/26; A61B 1/00009; A61B 1/0008;
A61B 1/00096; A61B 1/00194; A61B 1/063;
A61B 1/0676; A61B 1/07; A61B 1/313;
G01B 11/25; G02B 23/2423;** A61B 1/005;
A61B 1/05

(86) International application number:
**PCT/DK2017/050443**

(87) International publication number:
**WO 2018/113885 (28.06.2018 Gazette 2018/26)**

(54) **A 3D SENSOR SYSTEM COMPRISING AN OPTICAL TRANSMITTER DEVICE, A DETECTOR AND A COMPUTER SYSTEM**

3D-SENSORSYSTEM MIT EINER OPTISCHEN SENDERVORRICHTUNG, EINEM DETEKTOR UND EINEM COMPUTERSYSTEM

SYSTÈME DE CAPTEUR 3D COMPRENANT UN DISPOSITIF ÉMETTEUR OPTIQUE, UN DÉTECTEUR ET UN SYSTÈME INFORMATIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **20.12.2016 DK PA201671005**

(43) Date of publication of application:
**30.10.2019 Bulletin 2019/44**

(73) Proprietor: **Cilag GmbH International**
**6300 Zug (CH)**

(72) Inventors:
• **HANSEN, Steen Møller**
**2300 Copenhagen (DK)**
• **KIRKEGAARD, Henriette Schultz**
**1757 Copenhagen V (DK)**
• **LINDVOLD, Lars René**
**2980 Kokkedal (DK)**

• **HANSEN, André**
**2450 Copenhagen SV (DK)**

(74) Representative: **Plougmann Vingtoft a/s**
**Strandvejen 70**
**2900 Hellerup (DK)**

(56) References cited:
EP-A1- 3 056 934          WO-A1-2015/200712
WO-A2-00/42906          US-A- 3 941 121
US-A- 5 460 182          US-A- 5 605 532
US-A- 5 769 791          US-A- 5 845 634
US-A1- 2006 089 633      US-A1- 2008 064 925
US-A1- 2009 135 280      US-A1- 2010 010 313
US-A1- 2010 191 057      US-A1- 2011 009 694
US-A1- 2011 110 114      US-A1- 2013 066 304
US-A1- 2015 045 622      US-A1- 2015 196 193
US-A1- 2016 331 468      US-A1- 2016 341 951
US-B1- 6 475 226          US-B1- 6 485 413
US-B1- 6 503 195

**(Cont. next page)**

- TIMOTHY L PENNINGTON ET AL: "Miniaturized 3-D surface pro?lometer using a ?ber optic coupler", OPTICS & LASER TECHNOLOGY, 10 July 2001 (2001-07-10), pages 313 - 320, XP055282562, Retrieved from the Internet <URL:http://ac.els-cdn.com/S0030399201000238/1-s2.0-S0030399201000238-main.pdf?_tid=f4f724e2-384b-11e6-8dd2-00000aacb35f&acdnat=1466581136_e03ec401160aec960ac794e9cbfabfed> [retrieved on 20160622]
- SU WEI-HUNG ET AL: "Three-dimensional shape measurements using endoscopes", PROCEEDINGS OF SPIE/ IS & T, IEEE, US, vol. 9586, 26 August 2015 (2015-08-26), pages 95861H - 95861H, XP060060250, ISBN: 978-1-62841-730-2, DOI: 10.1117/12.2190221
- SCHICK ANTON ET AL: "3D measuring in the field of endoscopy", OPTICAL MEASUREMENT SYSTEMS FOR INDUSTRIAL INSPECTION VII, SPIE, 1000 20TH ST. BELLINGHAM WA 98225-6705 USA, vol. 8082, no. 1, 23 May 2011 (2011-05-23), pages 1 - 12, XP060013387, DOI: 10.1117/12.889167

**Description**

**TECHNICAL FIELD**

The invention relates to an optical transmitter device suitable for use in a 3D sensor system for determining 3D data within a body cavity. The invention also relates to a 3D sensor system comprising an optical transmitter device.

**BACKGROUND ART**

Minimally invasive surgery (MIS) and in particular laparoscopy has been used increasingly in recent years due to the benefits compared to conventional open surgery as it reduces the trauma to the patient skin and optionally further tissue, leaves smaller scars, minimizes post-surgical pain and enables faster recovery of the patient.

There are different kinds of MIS such as laparoscopy, endoscopy, arthroscopy and thoracoscopy. Whereas many of the MIS procedures are mainly for examination within natural openings of mammals, laparoscopy has in recent years developed to be a preferred method of performing both diagnostic and surgical procedures.

In laparoscopic surgery the surgeon accesses a body cavity, such as the abdominal or pelvic cavity, through a series of small incisions. A laparoscope is inserted through an incision, and conventionally connected to a monitor, thereby enabling the surgeon to see the inside of the abdominal or pelvic cavity. In order to perform the surgical procedure, surgical instruments are inserted through other incisions. In addition, the body cavity (surgery cavity) around the surgical site is inflated with a fluid, preferably gas e.g. carbon dioxide in order to create an 'air' space within the cavity to make space for the surgeon to view the surgical site and move the laparoscopic instruments.

Minimally invasive surgery is generally performed through openings in a patient's skin and the surgical site is visualized for the surgeon by inserting a laparoscope which comprises illumination means and a camera into the body cavity and displaying the images on a screen.

In order to improve the 3D surface determination for the surgeon, in particular to make it easier for the surgeon to determine the sizes of various organs, tissues, and other structures in a surgical site, several in-situ surgical metrology methods have been provided in the prior art. Different types of optical systems have been applied to provide an improved vision of the surgical site.

Also in other connections it may be difficult or expensive to obtain 3D surface data from internal body cavity surfaces of a patient. The Operator may for example use a CT scan to obtain the desired 3D surface data.

US 2013/0296712 describes an apparatus for determining endoscopic dimensional measurements, including a light source for projecting light patterns on a surgical sight including shapes with actual dimensional measurements and fiducials, and means for analyzing the projecting light patterns on the surgical site by comparing the actual dimensional measurements of the projected light patterns to the surgical site.

WO 2013/163391 describes a system for generating an image, which the surgeon may use for measuring the size of or distance between structures in the surgical field by using an invisible light for marking a pattern to the surgical field. The system comprises a first camera; a second camera; a light source producing light at a frequency invisible to the human eye; a dispersion unit projecting a predetermined pattern of light from the invisible light source; an instrument projecting the predetermined pattern of invisible light onto a target area; a band pass filter directing visible light to the first camera and the predetermined pattern of invisible light to the second camera; wherein the second camera images the target area and the predetermined pattern of invisible light, and computes a three-dimensional image.

US2008071140 discloses an endoscopic surgical navigation system which comprises a tracking subsystem to capture data representing positions and orientations of a flexible endoscope during an endoscopic procedure, to allow co-registration of live endoscopic video with intra-operative and/or preoperative scan images. Positions and orientations of the endoscope are detected using one or more sensors and/or other signal-producing elements disposed on the endoscope.

US2010268067 discloses methods, systems, devices, and computer-readable media for image guided surgery for allowing a physician to use multiple instruments for a surgery and simultaneously provide image-guidance data for those instruments.

US2011069159 discloses a system for orientation assistance and display of an instrument that is inserted or present in the natural or artificially produced hollow cavity (human, animal, object), and that is equipped with one or more sensor units. Multiple measurements of the 3D position of the instrument equipped with one or more sensor units are performed by positioning a measuring system, so that a precise orientation and positioning of the instrument in the body may be computed. The 3D position data are used to compute a virtual image of the instrument synchronously. The virtual images are then either projected directly in exact position onto the body surface of a person or combined in a body surface image (real video camera image of the patient) onto a monitor or superimposed (virtual or augmented reality).

It has also been suggested to generate augmented reality vision of surgery cavities for providing an improved view of internal structures of the organs of a patient to determine the minimal distance to a cavity surface or organ of a patient. Such systems are described in the articles "Augmented reality in laparoscopic surgical oncology" by Stéphane Nicolau et al. Surgical Oncology 20 (2011) 189-201 and "An effective visualization technique for depth perception in augmented reality-based surgical navigation" by Choi Hyunseok et al. The international journal of medical robotics and computer assisted surgery, 2015 May 5. doi: 10.1002/rcs.1657.

US20110110114 discloses a structured illumination surgical system comprising: a light source for providing a light beam; an optical cable, comprising an optical fiber, optically coupled to the light source for receiving and transmitting the light beam; a handpiece, operably coupled to the optical cable; an optical element, a proximal end of the optical element optically coupled to a distal end of the optical fiber, for receiving the light beam and scattering the light beam to illuminate a surgical site, wherein the surface area of the proximal end of the optical element is greater than the surface area of the distal end of the optical fiber.

EP3056934A1 discloses a measuring head of an endoscope inclusing projection optics for illuminating a specimen with a light pattern.

## DISCLOSURE OF INVENTION

An object of the present invention is to provide a device for enhancing 3D surface determination within a body cavity, such as a minimally invasive surgery cavity, which device is simple to use and at the same time may be used with a minimal discomfort and/or injury to a patient.

An object of an embodiment of the present invention is to provide an optical transmitter device for transmitting light within a body cavity which signal enhances the 3D determination for a surgeon.

An object of an embodiment of present invention is to provide an optical transmitter device for transmitting light within a body cavity which transmitter device is relatively low cost.

An object of an embodiment of the present invention is to provide an optical transmitter device for transmitting light within a body cavity for enhancing 3D surface determination.

An object of an embodiment of the present invention is to provide an optical transmitter device for use in a 3D sensor system for determining 3D data of a surface contour with high precision within a body cavity.

An object of an embodiment relates to a 3D sensor system comprising an optical transmitter device.

An object of an embodiment of present invention is to provide an optical transmitter device for transmitting light within a minimally invasive surgery cavity which device may be used with high flexibility.

An object of an embodiment of the present invention is to provide a 3D sensor system which is relatively simple to use, which may be used in a very flexible manner and/or which provides a surgeon with a very good intra cavity visibility prior to, during and/or after minimally invasive surgery.

One or more of these objects have been solved by the invention or embodiments thereof as defined in the claims or as described herein below.

It has been found that the invention or embodiments thereof have a number of additional advantages, which will be clear to the skilled person from the following description.

The invention is defined in the claims.

According to the invention the optical transmitter device comprises a penetrator member for penetrating through mammal skin for projecting a light pattern within a body cavity and an associated structured lightning. Thereby a device for enhancing 3D surface determination within the body cavity has been provided.

The optical transmitter device for emitting light within a body cavity comprises an elongate penetrator member for penetrating through mammal skin and an associated structured lightning member. The penetrator member has a distal portion and a proximal portion. The distal portion has a center axis and a length and comprises a penetrator tip. The structured lightning member comprises a light source and a projector, wherein the light source is operatively connected to the projector for delivering light to the projector. The projector is disposed at the distal portion of the penetrator member and is configured for projecting a diverging light pattern.

The penetrator tip is distally disposed at the distal portion of the penetrator member i.e. the penetrator tip at least in one position (referred to as its penetrating position) of the optical transmitter device forms the most distal part of the distal portion of the penetrator member.

Due to the shape of the distal portion of the optical transmitter device at least a part of the distal portion with the penetrator tip and including the projector can in a simple manner be introduced into the body cavity for enhancing 3D surface determination within the body cavity.

The penetrator tip can in a relatively simple manner be applied to penetrate the skin of a patient for being introduced into the body cavity while at the same time ensuring a minimal discomfort and/or injury to the patient. Further when withdrawing the distal portion of the optical transmitter device the opening in the skinn provided by the penetration will be relatively small and uncomplicated to close e.g. by using a patch. The penetration may even be so small that it closes itself due to the elasticity of the skin.

The term "body cavity" is herein used to denote any gas and/or liquid filled cavity within a mammal body. The cavity may be a natural cavity or it may be an artificial cavity which has been filled with a fluid (in particular gas) to reach a desired size. The cavity may be a natural cavity which has been enlarged by being filled with a fluid. Advantageously the body cavity is a minimally invasive surgery cavity.

The terms "distal" and "proximal" should be interpreted in relation to the orientation of the optical transmitter device or any other device used in connection with minimally invasive surgery.

The phrase "distal to" means "arranged at a position in distal direction to the optical transmitter device, where the direction is determined as a straight line between a proximal end of the optical transmitter device and the distal end of the optical transmitter device. The phrase "distally arranged" means arranged distal to the distal end of the optical transmitter device.

The phrase "real time" is herein used to mean the time it requires the computer to receive and process optionally changing data optionally in combination with other data, such as predetermined data, reference data, estimated data which may be non-real time data such as constant data or data changing with a frequency of above 1 minute to return the real time information to the operator. "Real time" may include a short delay, such as up to 5 seconds, preferably within 1 second, more preferably within 0.1 second of an occurrence.

The term "operator" is used to designate a human operator (human surgeon) or a robotic operator i.e. a robot programmed to perform a minimally invasive diagnostic or surgery procedure on a patient. The term "operator" also includes a combined human and robotic operator, such as a robotic assisted human surgeon.

The term "access port" means a port into a body cavity provided by a cannula inserted into an incision through the mammal skin and through which cannula an instrument may be inserted. The term "penetration hole" means a hole through the mammal skin without any cannula.

The term "rigid connection" means a connection which ensures that the relative position between rigidly connected elements is substantially constant during normal use.

The term "cannula" means herein a hollow tool adapted for being inserted into an incision to provide an access port as defined above.

Often the surface of the minimally invasive surgery cavity is much curved. The term 'target area' of the surface of the minimally invasive surgery cavity is herein used to designate an area which the surgeon has focus on, e.g. for diagnostic purpose and/or for surgical/interventional purpose.

The term "skin" is herein used to designate the skin of a mammal. As used herein the skin may include additional tissue which is or is to be penetrated by the penetrator tip.

The term "about" is generally used to include what is within measurement uncertainties. When used in ranges the term "about" should herein be taken to mean that what is within measurement uncertainties is included in the range.

It should be emphasized that the term "comprises/comprising" when used herein is to be interpreted as an open term, i.e. it should be taken to specify the presence of specifically stated feature(s), such as element(s), unit(s), integer(s), step(s) component(s) and combination(s) thereof, but does not preclude the presence or addition of one or more other stated features.

Throughout the description or claims, the singular encompasses the plural unless otherwise specified or required by the context.

A light pattern or pattern of light includes a group of electromagnetic waves projected from the projector and propagating along parallel or diverging directions and wherein the light is textured seen in a cross-sectional view orthogonal to a center axis (herein also referred to as the optical axis) of the group of electromagnetic waves i.e. the light has areas of higher intensity, and areas of lower intensities or no intensity which is not a naturally intensity distribution of a light beam. The terms "light pattern" and light texture" is used interchangeable.

The projector may have one fixed position or it may be movably between two or more positions. Advantageously the projector has at least one position wherein it is adapted for projecting the light pattern outwards relative to the optical transmitter device, preferably for projecting the light pattern distally relative to the optical transmitter device.

In an embodiment at least the distal portion of the penetrator member may be shaped as a needle with the penetrator tip at its distal end.

To ensure a very low discomfort and/or injury to a patient the distal portion of the penetrator member advantageously has an average cross-sectional dimension of up to about 1 cm, such as up to about 5 mm or even less.

In an embodiment the distal portion penetrator member advantageously has an average cross-sectional dimension of up to about 3 mm.

The cross-sectional dimensions of the distal portion are determined perpendicular to the center axis of the distal portion.

In an embodiment the distal portion of the penetrator member has a maximal cross-sectional dimension of up to about 1 cm, such as up to about 5 mm, such as up to about 3 mm, such as up to about 2 mm.

Advantageously the maximal cross-sectional dimension of the penetrator member is up to about 1 cm, such as up to about 5 mm or even less. The very small cross sectional size of the penetrator member makes it simpler for the operator to introduce at least a part of the distal portion of the penetrator member into the body cavity with minimal discomfort or injury of the patient.

In order to have a simple penetration of the skin and introduction of at least a part of the distal portion of the penetrator member into the body cavity it has been found that the average and/or maximal cross sectional dimension of the distal portion of the penetrator member advantageously should be relatively narrow and thus it was found that the light beam from the light source will be relatively small. By ensuring that the projector projects the light pattern with a diverging angle having a cross sectional periphery which is relatively large at a relatively short distance from the projector may be provided.

Advantageously the projector is configured for projecting the light pattern with a diverging angle of at least about 10 degrees, such as at least about 20 degrees, such as at least about 30 degrees, such as at least about 40 degrees relative to the optical axis of the light pattern. Preferably the diverging angle is selectable, e.g. by being tunable by an operator.

The diverging angle is advantageously determined as the beam divergence, Θ:

$$\Theta = \left(2 \, arctan \begin{pmatrix} \frac{1}{2}D2 - \frac{1}{2}D1 \\ 2L \end{pmatrix}\right)$$

wherein D1 is the largest cross-sectional dimension orthogonal to the center axis of the light pattern a first distance from the projector, D2 is the largest cross-sectional dimension orthogonal to the center axis of the light pattern a second larger distance from the projector and L is the distance between D1 and D2 and wherein the distances is determined along the center axis of the light pattern.

A desired length of the distal portion of the penetrator member depends largely on the intended use of the optical transmitter device and in particular the size of the body cavity and the thickness of the skin to be penetrated to reach into the body cavity. Choosing a too long length of the distal portion of the penetrator member may increase the risk of inserting the distal portion of the penetrator member too long into the body cavity and thus the risk of damaging an inner surface area of the body cavity. Choosing a too short length of the distal portion of the penetrator member may increase the risk that the distal portion of the penetrator member is not sufficiently long to enter into the body cavity, e.g. in obese patients.

In an embodiment the distal portion has a length of at least about 0.5 cm, such as at least about 1 cm, such as from about 1 cm to about 30 cm, such as up to about 20. In an embodiment the length of the distal portion of the penetrator member is selectable by an operator. The length of the distal portion of the penetrator member may for example be adjustable by the surgeon e.g. by comprising a telescopic adjustable length section and/or comprising an obstruction that may be adjusted.

In an embodiment length of the distal portion of the penetrator member is selectable and the penetrator member comprises an obstruction arranged to define a boundary between the distal portion and the proximal portion of the penetrator member and for preventing intrusion of the proximal portion into the skin, preferable the obstruction being displaceable, e.g. by an operator. The obstruction may for example be a flange and or a neck protruding from the penetrator member and advantageously is displaceable along the length section of the penetrator member.

In principle, the penetrator member may have any cross-sectional shape, however generally it is desired that it does not have sharp edges which unintentionally may damage the skin of the patient. Advantageously at least a length section of the distal portion has a substantially circular, oval, crescent or semi-circle cross-sectional periphery. Preferably the entire length of the distal portion has a substantially round, oval, crescent or semi-circle cross-sectional periphery. The entire length of the distal portion of the penetrator member is determined from the proximal portion to the tip.

Where the optical transmitter device is adapted to be rotationally moved after penetrating the skin, it is desired that the distal portion of the penetrator member has a substantially circular cross-sectional shape to thereby reduce the risk of unintendedly damaging the skin of the patient by the rotational movement of the optical transmitter device.

In an embodiment the distal portion comprises an angular cross-sectional periphery at least in a length section or in the entire length from its tip and towards the distal portion. Optionally the distal portion at least from its tip and towards the distal portion comprises a substantially triangular quadrangular pentagonal or hexagonal cross-sectional periphery. The angular cross-sectional periphery of the distal portion of the penetrator member may make it simpler to introduce the distal portion of the penetrator member into the skin and the body cavity where the skin has a relatively low elasticity e.g. skin of older patients.

Advantageous at least a part of the distal portion of the penetrator member - optionally the entire distal portion of the penetrator member - is rigid to ensure a relatively simple penetration. In an embodiment at least the penetrator tip is at least partly of metal, such as steel. In an embodiment the entire distal portion of the penetrator member is of metal, such as steel.

For ensuring a simple insertion of the optical transmitter device into the body cavity it is generally desired that the distal portion of the penetrator member is either straight or not too curved.

In an embodiment the distal portion of the penetrator member is substantially straight.

In an embodiment the distal portion of the penetrator member comprises a curvilinear length section, such as a curvilinear length section having a curvature radius of up to about 90 degrees, such as up to about 60 degrees, such as up to about 30 degrees, advantageously the curvature radius of the distal portion of the penetrator member is selectable by an operator.

The penetrator tip advantageously has a relatively sharp apex and/or a relatively sharp edge for ensuring a simple penetration of the skin with a minimum discomfort and/or injury to the patient.

In an embodiment the penetrator tip is selected from a conical shaped tip, a triangular shaped tip, a blunt shaped tip or a bevel shaped tip. The penetrator tip is for example a bevel shaped tip having a bevel angle of at least 10 ° to the axis of the distal portion of the penetrator, such as from about 15° to about 85°. In an embodiment the penetrator tip comprises an apex and/or a cutting edge.

The projector is adapted to receive the light from the light source directly, via free space transmission and/or via a waveguide and shaping the received light to a textured light. The projector comprises a diffractive optical element (DOE).

The diffractive optical element may be an optical component comprising a microstructured surface. When a DOE is placed in front of a light beam it reshapes the light beam into a pattern. The pattern of the light, diffracted by a grating, depends on the structure of the elements. These patterns may be tailored to the application requirements. A DOEs generally creates pattern(s) through diffraction. The larger the output pattern, the smaller the features which may be required to create it. Feature sizes also scale with wavelength. Thus, in order to make diffractions for use with shorter wavelengths, smaller feature sizes may be patterned.

The DOE is advantageously a glass based DOE.

In an embodiment the light source is operatively connected to the projector by a light guiding structure, e, g. comprising a waveguide, such as a light guiding structure comprising an optical fiber. Generally it is desired to use an optical fiber for ensuring a high confinement and control of the light.

In an embodiment the projector comprises a diffractive optical element and the light guiding structure comprises an optical fiber with a light output end configured for transmitting an output light beam towards the diffractive optical element. Such an arrangement of optical fiber and DOE can be relatively compact.

Advantageously the light guiding structure and the projector forms part of a projector probe. The projector probe comprises a beam expanding lens arrangement arranged prior to or after the projector, wherein "prior to" and "after is to be determined based on the light propagating direction.

In an embodiment the projector probe is a disclosed in the co-pending application PA 2017 70631 DK.

The beam expanding lens arrangement comprises a gradient index (GRIN) lens. The beam expanding lens arrangement ensures that the beam of light reaching the DOE is sufficient large in beam size to ensure a diverging light pattern i.e. a light pattern that is spreading as it propagates to reach a light pattern cross sectional periphery which is relatively large at a relatively short distance from the projector.

Due to the beam expanding lens arrangement even a very narrow light beam from the light source can be expanded to a diverging light pattern with a cross sectional periphery which is relatively large at a relatively short distance from the projector. Thus, the average and maximal cross sectional dimension of the distal portion of the penetrator member may be very narrow, such as less than 5 mm, such as less than 3mm or even less than 2 mm to ensure an easy penetration through the skin of the patient with a minimum discomfort and/or injury to the patient.

In an embodiment the light guiding structure is an optical fiber having a light output end and the projector probe comprises the optical fiber light output end and the DOE in a fixed and/or operator controlled distance relative to each other.

Generally, it is desired that the beam expanding lens arrangement is arranged between the optical fiber light output end and the DOE in order to have the DOE operating as effective as possible.

In an embodiment the beam expanding lens arrangement is tunable.

Due to the slender shape of the distal portion of the optical transmitter device and the distal portion of the penetrator member thereof the beam from the light source is as described relatively small and thus in an embodiment the light guiding structure has a beam output with a beam output spot, of at least about 4 $\mu$m, such as at least about 8 $\mu$m, such as at least about 10 $\mu$m, such as at least about 12 $\mu$m, the beam output is preferably single mode.

The spot diameter is determined as the average spot diameter. Further it should be noted that unless otherwise stated or clear from the contest, the diameter of the light pattern, light beam, light spot, beam diameter or similar is determined as the average diameter unless otherwise specified or clear from the context.

Advantageously the light source is operatively connected to the projector such that a beam of light impinges onto the projector. To ensure an effective formation of the pattern it has been found that the impinging light beam should have a diameter which is relative large. In an embodiment the impinging beam of light has a beam diameter of at least about 0.4 mm, such as at least about 0.6 mm, such as at least about 0.7 mm.

The projector is advantageously disposed at or sufficiently near to the penetrator tip to be introduced into the body cavity when the optical transmitter device is in use. Thus the thickness of the skin to be penetrated and the size of the body cavity may influence at which position at the distal portion of the penetrator member the projector may be disposed to be fully operable. Advantageously the projector is disposed at the distal portion of the penetrator member at a distance from the proximal portion of the penetrator member which is larger than the thickness of the skin to be penetrated to reach the body cavity. As mentioned above the projector may form part of a probe e.g. the projector may form a distal portion of the probe.

In an embodiment the projector is disposed within a distance to the penetrator tip of up to about 80% of the length of the distal portion of the penetrator member, such as up to about 60% of the length of the distal portion of the penetrator member, such as up to about 40% of the length of the distal portion of the penetrator member, such as up to about 30% of the length of the distal portion of the penetrator member.

In an embodiment the projector is disposed at the penetrator tip or within a distance to the penetrator tip of up to about 15 cm, such as up to about 10 cm, such as up to about 5 cm, such as up to about 1 cm, such as up to about 0.5 cm from the penetrator tip determined along the length of the distal portion of the penetrator member.

The projector (e.g. in the form of the entire projector probe) may be permanently or releasable fixed to the distal portion of the penetrator member. In an embodiment the projector is fixed to a coating or to a sleeve fixed to the distal portion, In an embodiment the projector and advantageously the entire projector probe may be removed from the penetrator member after use of the optical transmitter device, such that the penetrator member may be cleaned e.g. by being subjected to a disinfection and/or sterilization procedures.

Generally, it is desired that the projector and the distal portion of the penetrator member is connected to each other when the optical transmitter device is in or adapted for use. Thus, in use it is desired that the penetrator and the projector cannot be independently removed from a patient, without also removing other part. Thereby the handling of the optical transmitter device becomes simpler and safer for the patient.

In an embodiment the projector (e.g. in the form of the projector probe) is fixed directly to the distal portion of the penetrator member.

In an embodiment the penetrator member comprises a lumen and the projector (e.g. in the form of the projector probe) is disposed in the lumen. Preferably, the diameter of the lumen corresponds to the max diameter of the projector/projector probe, preferably such that the diameter of the lumen is at most 2 mm larger than the max diameter of the projector probe. The lumen preferably forms part of a channel extending in and along the length of the distal portion of the penetrator member. Thereby the projector may be protected by the penetrator member during the introduction through the skin and into the body cavity. Further it may be ensured that the projector during the penetration step does not protrude orthogonally from the penetrator member, i.e. it is within the outer periphery of the penetrator member or it is aligned with the penetrator member which reduces or removes any risk of damaging the patient by the projector.

In an embodiment the lumen or the channel including the lumen is substantially centrally positioned relative to the axis of the distal portion of the penetrator member. For minimizing the diameter of the distal portion of the penetrator this embodiment is very advantageous.

In an embodiment the lumen or the channel including the lumen is non-centrally positioned relative to the axis of the distal portion of the penetrator member, i.e. the channel/lumen may be positioned radially outwards relative to the axis of the penetrator member. Preferably the channel/lumen is extending substantially along the axis with a distance to the axis, such as a distance of from about 1 mm to about 35 % of the cross-sectional diameter of the penetrator member, such as up to 25 % of the cross-sectional diameter of the penetrator member, such as with a distance up to about 3 mm, such as a distance up to about 1 mm to the axis.

In an embodiment wherein the projector (e.g. in the form of the projector probe) is disposed in the lumen, the lumen may advantageously be open or openable at the penetrator tip, e.g. by comprising a removable paste and/or a lid. Thereby the projector may be passed through the opening after the penetrator member has been partly introduced into the body cavity. The projector may thereby be protected against dust or other undesired contaminations outside the body cavity. The risk of infecting the patient may thereby be reduced.

Advantageously the projector is pivotable and/or displaceable along the axis of the distal portion of the penetrator member. Thereby the projector may be displaced or pivoted into operating position after having introduced the distal portion of the penetrator member sufficiently into the body cavity. The projector may for example be fixed to be pivotably folded from a first covered position to a second projecting (operating) position and/or the projector may be fixed to be axially displaced from a first retracted position to a second projecting position ready for projecting the light pattern within the body cavity e.g. towards a target surface area within the body cavity. Still it is preferred that the penetrator member and the projector is inseparable when the optical transmitter device is in use. After use the various parts of the optical transmitter device may be separated from each other e.g. for cleaning purpose.

In an embodiment the projector is fixed to the distal portion of the penetrator member such that it has a first folded position where the projector is folded into the penetrator member to be covered and protected by the penetrator member and the projector has a second unfolded and/or pivoted projecting position where it is in position for projecting the light pattern. The optical transmitter device is adapted for penetrating into the body cavity when the projector is in its first folded position. After the penetrator tip has penetrated the skin and at least a part of the distal portion of the penetrator member has been introduced into the body cavity, the projector is unfolded and/or pivoted to its second unfolded and/or pivoted projecting position within the body cavity and the projector is now ready for projecting the diverging light pattern within the body cavity.

In an embodiment the optical transmitter device comprises a tip protecting arrangement configured for fully or partly covering the tip after the distal portion of the penetrator member has penetrated the skin and entered into a body cavity. The tip protecting arrangement for example comprises a flange optionally forming part of the projector and/or the projector probe.

Since the penetrator tip may be (and advantageously is) relatively sharp, it is advantageous that the penetrator tip is at least partly covered when it is inside the body cavity such that the risk of undesired damage of tissue by the penetrator tip within the body cavity may be reduced or even avoided.

In an embodiment the optical transmitter device comprises an intra cavity protecting arrangement for protecting against undesired impacts by the penetrator tip within the body cavity. The intra cavity protecting arrangement is advantageously configured for switching the penetrator tip between a first penetrator position (where the penetrator tip is adapted for penetrating the skin into the body cavity) and a second penetrator tip protected position. In the second penetrator tip protected position the penetrator tip may e.g. be retracted, unfolded and/or covered.

In an embodiment the intra cavity protecting arrangement comprises an axial displacement arrangement for withdrawing and optionally removing at least the penetrating tip of the distal portion of the penetrator member. The projector and/or projector probe may for example be arranged in a cannel (lumen) of the distal portion of the penetrator member and the axial displacement arrangement is configured for removing a part of the distal portion of the penetrator member covering the projector to thereby exposing the projector. The removable part of the penetrator member may comprise the penetrator tip.

In an embodiment the intra cavity protecting arrangement comprises an axial displacement arrangement comprising a slidable tip cover having the first penetrator position such that the penetrator tip is exposed and having the second penetrator tip in a protected position such that the slidable tip cover covers the penetrator tip. The slidable tip cover may be in its second penetrator tip protected position when unloaded. Thereby the penetrator tip will be covered unless it is activated/released or in other way loaded to become in its first penetrator position. Thus, the penetrator tip may advantageously automatically be in its second penetrator tip protected position when it is not to penetrate the skin of a patient. Optionally the slidable tip cover is tube shaped and preferably coincident with the distal portion of the penetrator member. The slidable tip cover may be arranged to surround the penetrator tip to set the penetrator tip in its second penetrator tip protected position.

In an embodiment the intra cavity protecting arrangement comprises an axial displacement arrangement for partly withdrawing at least the penetrating tip of the distal portion of the penetrator member.

The projector and/or projector probe may for example be arranged in a channel of the distal portion of the penetrator member and the axial displacement arrangement is configured for axially displacing of the distal portion of the penetrator member and thereby the penetrator tip to thereby exposing the projector.

In an embodiment the projector and/or projector probe is arranged in a sleeve fixed to surround the distal portion of penetrator member and the axial displacement arrangement is configured for axially displacing (e.g. by partly withdrawing) of the distal portion of the penetrator member or axially displacing the sleeve, such that the penetrator tip will be surrounded by the sleeve for providing the protection against undesired impacts by the penetrator tip within the body cavity. Advantageously the sleeve forms a part of or the entire slidable tip cover. To ensure a safe penetration the sleeve is advantageously of a rigid material.

In an embodiment the intra cavity protecting arrangement comprises a pivotable and/or displaceable flange which can be pivoted and/or displaced to fully or partly cover the penetrating tip for providing the protection against undesired impacts by the penetrator tip within a body cavity.

In an embodiment the penetrator tip is foldable from the first penetrator position to a second unfolded position. The penetrator tip is advantageously in the penetrator tip protected position when in its unfolded position. For example the penetrator tip may be folded back in one or preferably 2, 3 or more penetrator tip leaf sections for providing the protection against undesired impacts by the penetrator tip within the body cavity.

In an embodiment the intra cavity protecting arrangement is configured for holding the penetrator tip in its second penetrator tip protected position when unloaded (e.g. prior to penetration and when in body cavity) and to switching to its first penetrator position when it is adapted for being penetrating the skin into the body cavity. The penetrator tip may advantageously be switched to its first penetrator position upon activation by an operator and/or upon activating by application of force to the penetrating tip. The activation may advantageously be provided by application of proximally directed force to the penetrator tip.

In order to ensure a penetration of the skin with a minimum discomfort and/or injury to the patient the penetrator tip should advantageously be relatively sharp as described above. However, also the shape of the remaining part of the distal portion of the penetrator member should advantageously be slender and without undesired protruding elements. In an embodiment the distal portion of the penetrator member and the projector forms a needle adapted for penetrating mammal skin for intra cavity positioning of the penetrating tip. The penetrator tip thereby forms a needle tip, preferably with a sharp apex. The optical transmitter device advantageously has a penetrating state, where the distal portion of the penetrator member and the projector forms the slender needle body and a projecting state, wherein at least one part of the optical transmitter device is pivoted and/or displaced and/or unfolded to fully or partly cover the penetrating tip, to fully or partly remove the penetrating tip and/or to positioning the projector for projecting the light pattern outwards relative to the optical transmitter device, preferably for projecting the light pattern distally relative to the optical transmitter device.

The light pattern (also referred to as textured light), comprises structured light comprising a symmetrical or an asymmetrical light pattern. In an embodiment the light pattern comprises a plurality of light dots e.g. arranged in with a repeating pattern or in a random configuration wherein the various dots may be equal or different in size, intensity and/or shape.

In an embodiment the light pattern comprises an arch shape, ring or semi-ring shaped lines, a plurality of angled lines and/or a coded structured light configuration. Preferably, the pattern comprises a grid of lines, a crosshatched pattern optionally comprising substantially parallel lines. The light pattern shape and structure is determined in a propagation plan perpendicular to the propagation axis (center axis) of the light pattern. In an embodiment the light pattern comprises a combination comprising two or more of the above mentioned pattern structures. For example the light pattern may comprise dots and angular lines in combination or dots in combination with a grid or triangular structures.

In an embodiment the structured light comprises areas of light and areas of no-light and/or areas of light of a first quality of a character and areas of light of a second quality of the character, wherein the character(s) advantageously is/are selected from light intensity, wavelength and/or range of wavelengths.

Advantageously the projector is configured for generating the light pattern by shaping the light received directly (with or without free space transmission) or via one or more other optical elements from the light source. The light pattern may advantageously be tunable e.g. by being selectable e.g. by the operator. The light pattern may e.g. be tunable with respect to light intensity, wavelength, range of wavelengths, bandwidth, shape, divergence and/or modulation frequency.

The optical transmitter device is advantageously adapted to be operated from its proximal portion. The proximal portion of the penetrator member is not adapted to be introduced into the skin or the body cavity of the patient and may comprise projecting element(s).

In an embodiment the proximal portion of the penetrator member comprises an operating member for being operated by an operator, such as a handle for being operated by a surgeon or a connector for being connected to or integrated with a robot for robotic operation. When the optical transmitter device is for manually use by a human surgeon the optical transmitter device advantageously has a handle such that it is usually known from minimally invasive surgery instruments.

When the optical transmitter device is adapted to be operated by a robot the proximal portion of the penetrator member is advantageously adapted to the robot or even integrated with the robot. For simple cleaning and/or replacement of the optical transmitter device or part(s) thereof it is desired that the proximal portion of the penetrator member of the optical transmitter device is releasable mounted to the robot, preferably comprising a rigid mount. The rigid mount may e.g. comprise or consist of a mechanical lock and/or a magnetically lock. The proximal portion of the penetrator member may be telescopic to provide a desired and selectable length or for assist in the operation -e.g. the penetration procedure - of the optical transmitter device.

In an embodiment one or more setting and/or one or more operations and/or one or more selections and/or one or more tunings are performable by an operator via the proximal portion of the penetrator member.

The light source may in the broadest aspect of the optical transmitter device be any kind of light source capable of generating sufficient light for the light pattern.

The light source may be a coherent light source or an incoherent light source. Examples of light sources includes a semiconductor light source, such as a laser diode and/or a VCSEL light source as well as any kind of laser sources including narrow bandwidth sources and broad band sources.

It is generally preferred that the light source is a laser light source.

The determination of light, including wavelengths, bandwidth, shape and similar is based on full width at half maximum (FWHM) determination unless otherwise specified or clear from the context.

In an embodiment the light source is a fiber laser and/or a semiconductor laser, the light source preferably comprises a VCSEL or a light emitting diode (LED).

In an embodiment the light source is adapted for emitting modulated light, such as pulsed or continuous-wave (CW) modulated light, preferably with a frequency of at least about 200 Hz, such as at least about 100 KHz, such as at least about 1 MHz, such as at least about 20 MHz, such as up to about 200 MHz or more.

The light source may be a coherent light source or an incoherent light source. For providing a sharp and controllable pattern within a narrow space it is generally desired that the light source is a laser type light source which is normally considered to be a substantially coherent light source.

The wavelength or wavelengths may in principle comprises any wavelengths, such as from the low UV light to high IR light e.g. up to 3 my or larger.

In an embodiment the light source is configured for emitting at least one electromagnetic wavelength within the UV range of from about 10 nm to about 400 nm, such as from about 200 to about 400 nm.

In an embodiment the light source is configured for emitting at least one electromagnetic wavelength within the visible range of from about 400 nm to about 700 nm, such as from about 500 to about 600 nm.

In an embodiment the light source is configured for emitting at least one electromagnetic wavelength within the IR range of from about 700 nm to about 1 mm, such as from about 800 to about 2500 nm.

The band width of the light source may be narrow or wide, however often it is desired to use a relatively narrow wavelength for cost reasons and optionally for being capable of distinguishing between light emitted from or projected from different elements e.g. from an optical transmitter device of an embodiment of the invention and from an endoscope or from two different optical transmitter devices.

In an embodiment the light source has a band width of up to about 50 nm, such as from 1 nm to about 40 nm.

In an embodiment the light source has a band width of which is larger than about 50 nm, such as a supercontinuum band width spanning over at least about 100 nm, such as at least about 500 nm. Where the light source has a large bandwidth, the optical transmitter device may advantageously comprise an optical filter e.g. a tunable filter such that the wavelength(s) of the light pattern may be selected.

The optical transmitter device may include two or more light sources which may operate with equal or different bandwidths, wherein the two or more.

In an embodiment the structured lightning member comprises two or more light sources having equal or different bandwidths, wherein the two or more pattern light sources operatively connected to the projector.

The power of the light source may be selected in dependence of the intended use of the optical transmitter device. Generally it is desired to apply a power source with a relative low power to protect against overheating, in particular where the optical transmitter device may be used for linger time e.g. for more than 10 minutes, e.g. for more than 30 minutes in a row. It may be advantageous that the power source is power tunable; thereby the power may e.g. be shortly increased to remove optional fog or mist on the projector. Alternatively a heating element may be provided to remove or prevent fog or mist on the projector.

Fog or mist may for example form when inserting the optical transmitter device into the body cavity. Due to the relatively low temperature of the projector, higher temperature and/or humidity in the body cavity mist /fog may form on the projector.

In an embodiment the optical transmitter device comprises a heating element arranged for heating the front face (distally face) of the projector to remove or prevent the formation of fog/mist.

In an embodiment at least a part of the projector - e.g. a front surface layer of the projector - comprises light absorbing elements or ions e.g. as described further below in connection with the description of the probe. In an embodiment the projector comprises an optical filter e.g. as described further below in connection with the description of the probe.

The light source may for example configured for emitting light at a power of from about 0.1 mW to about 100 mW, such as from about 1 mW to about 50 mW, such as from about 3 mW to about 5 mW. Advantageously the optical transmitter device comprises a regulator for tuning the light source power.

The optical transmitter device advantageously comprises a computer operatively connected to the light source, the projector and/or at least one element of the optical transmitter device, such as the light source, or an optical filter or lens. The computer may be any kind of processor operating element or system. The computer may advantageous be adapted for fully or partly operating the light source, the projector and/or the at least one additional element of the optical transmitter device.

The invention also relates to a 3D sensor system comprising an optical transmitter device as described herein and an associated detector. The associated detector is adapted for detecting reflected rays of the light pattern projected by the projector of the optical transmitter device.

The associated detector may in principle be any kind of detector capable of sensing at least one wavelength of the light projected by the projector.

Advantageously the associated detector is a camera comprising an array of pixel sensors. Each of the pixel sensors preferably comprises a photodetector, such as an avalanche photodiode (APD), a photomultiplier or a metal-semiconductor-metal photodetector (MSM photodetector). The pixel sensor may advantageously include one or more active pixel sensors (APS).

Each pixel sensor may comprise an amplifier. The associated detector may advantageously comprise at least about 1 kilo pixels, such as at least about 1 Mega pixels.

In an embodiment the associated detector is selected from a charge-coupled device (CDD) image sensor, or a complementary metal-oxide-semiconductor (CMOS) image sensor.

In an embodiment the associated detector is a mono detector.

In an embodiment the associated detector is a stereo detector.

The associated detector may advantageously comprise a band pass filter for suppressing back-ground light, such as light with wavelength not included in the projected light pattern. Thereby the associated detector may be adapted for distinguishing between light originating from the projected light pattern and light from another source, such as from an endoscope or from a minimally invasive surgery instrument as described in WO 2015/124159.

The associated detector may advantageously comprise at least one aperture lens, such as a Fresnel lens for collecting reflected light.

The associated detector may be disposed on or integrated with any instrument adapted for being introduced into the body cavity. For example the associated detector may be disposed on or integrated with a cannula, a scope of a surgical intervention instrument and/or a sensor penetrator.

In an embodiment the associated detector is an endoscope, such as a laparoscope, such as a mono endoscope or a stereo endoscope.

In an embodiment the associated detector is disposed on or integrated with a sensor penetrator. The sensor penetrator may comprise a sensor penetrator tip adapted for penetrating mammal skin for intra cavity positioning of the detector. The sensor penetrator tip may be as the penetrator tip of the optical transmitter device as disclosed herein.

Advantageously the 3D sensor system comprises a computer system. The computer system may comprise a single computer or several data interconnected computers and digital storing device(s) e.g. including one or more databases. The computer system advantageously includes at least one digital, programmable electronic device adapted for perform arithmetic and logical operations at high speed.

Advantageously the computer system is operatively connected to the optical transmitter device and the associated detector and being configured for calculating data representing a 3D surface contour of a surface area reflecting light transmitted from the optical transmitter device based on 3D signal data from the optical transmitter device and detected data from the detector. The optical transmitter device may advantageously comprise a spatially and/or orientation detector for detecting the spatial and/or orientation of the projector e.g. relative to a fixed point. Also the associated detector advantageously comprises a spatially and/or orientation detector for detecting the spatial and/or orientation of the projector e.g. relative to a fixed point e.g. the same fixed point used for reference to the projector. In an embodiment wherein the optical transmitter device and optionally the associated detector is/are connected to a robot the spatially and/or orientation detector for detecting the spatial and/or orientation of the projector/detector may form part of the robot.

In an embodiment a computer system is configured for receiving optical transmitter device position data and for using the optical transmitter device position data in the calculation of the 3D surface contour. The optical transmitter device position data is optionally fed to the computer system by an operator and/or the optical transmitter device comprises a positioning sensor determining the optical transmitter device position data of the optical transmitter device. The optical transmitter device position data preferably comprises the spatial position of the projector and a propagation direction of the light pattern.

Advantageously the computer system is configured for receiving detector position data and using the detector position data in the calculation of the 3D surface contour. The detector position data is optionally fed to the computer system by an operator and/or the detector comprises a positioning sensor determining the detector position data of the associated detector. The detector position data preferably comprises the spatial position and the orientation of the detector.

In an embodiment the computer system is configured for receiving angular position data representing the angle between the optical axis of the projector and the optical axis of the associated detector and/or the computer system is configured for receiving data for calculating the angular position data. In an embodiment the angle between the optical axis of the projector and the optical axis of the associated detector are fixed or the angle may be set to a fixed degree size. The optical axis means the center axis of projected or detected light (FWHM).

In an embodiment the optical transmitter device and the associated detector are interconnected to set and/or to determine the angular position data. The optical transmitter device and the associated detector may e.g. be interconnected to define the angle between the optical axis of the projector and the optical axis of the associated detector. Thereby the optimal angle for detection of reflected light and for calculation of the 3D surface contour may be ensured. The angle may for example be selectable by the operator. In an embodiment the interconnection between the optical axis of the projector and the optical axis of the associated detector is established by an interconnecting arrangement fixed to respectively the proximal portion of the penetrator member and to a proximal portion of the associated detector.

Advantageously - to obtain a high quality resolution it has been found that the structured light projector and the camera should have angles relative to the tissue plane of maximum 60 degrees. Thus, it has been found that the longitudinal axis of projector and camera should be at maximum 60 degrees at the intersection point. Preferably, the angle between the optical axis of the projector and the optical axis of the associated detector is between about 5 and about 40 degrees, such as between about 5 and about 35 degrees.

In use, it is generally desired that the projector is positioned about 1 to about 30 cm from the target surface area, preferably about 3 to about 10 cm from the target surface area. The associated sensor is advantageously positioned about 1 to about 30 cm from the target surface area, preferably about 3 to about 10 cm from the target surface area.

Advantageously the 3D sensor system forms part of an operator in the form of a robot, i.e. one or more of the elements of the 3D sensor system are fixed to or integrated with the robot. Preferably, both the transmitter device and the associated detector are connected to and/or are integrated with the robot. Thereby the angular position data may be retrievable by the robot and preferably the computer system forms part of or are in data connection with a computer of the robot.

In an embodiment the 3D sensor system is configured for real time operation and the computer system is advantageously configured for real time calculating of data representing the 3D surface contour.

In an embodiment the computer system is configured for calculating data representing a 3D reconstruction of at least a part of a body cavity.

The reconstruction technique may e.g. include point cloud surface reconstruction. A point cloud is a set of data points in a three-dimensional coordinate system, these points may be defined by X, Y, and Z coordinates, intended to represent the external surface of interest. The reconstruction technique from the data transmitted to the computer system may e.g. be as or correspond to the 3D reconstruction technique as described in "A low cost 3D scanner based on structured light" by C. Rocchihi et al.

Eurographics vol. 20 (2001), number 3.

In an embodiment the computer system is advantageously further in data communication with or comprises a database for receiving pre operation data and for using such pre operation data in its calculation.

The pre operation data may in principle be any data that represents at least one attribute of the body cavity, such as at least one dimension, size or relative size. In an embodiment the pre operation data is data stored on a database and which is estimated and/or calculated for groups of patient. The pre operation data may thus be retrieved from the database based on one or more characteristic of the patient, such as age, gender and/or weight of the patient. In an embodiment the pre operation data is data obtained from an actual measurement of the patient, such as an imaging and/or a scanning e.g. CT scanning, MR scanning, ultra sound scanning or another form of scanning.

In an embodiment the computer system is configured for acquiring the pre operation data, optionally the computer system comprises the database that stores the pre operation data. In an embodiment the computer system comprises an interface for receiving the pre operation data or for receiving information about how to select the pre operation data from the database.

In an embodiment the computer system is configured for receiving intra operation data representing at least one attribute of the body cavity during the minimally invasive surgery and/or diagnostic procedure and for using said intra operation data for calculating data representing a 3D reconstruction of at least a part of a body cavity. The intra operation data may be any kind of data representing at least one attribute of the body cavity, such as at least one dimension, size or relative size. The intra operation data differs from the pre operation data in that the intra operation data is obtained during the minimally invasive surgery and/or diagnostic procedure whereas the pre operation data is obtained prior to performing the minimally invasive surgery and/or diagnostic procedure, is estimated and/or is calculated data based on pre operation data such a ultrasound data or data generated from measurements of the body cavity obtained using ultrasound..

In an embodiment the intra operation data comprises data derived from measurements (e.g. a CT/MR scanning) obtained during the minimally invasive surgery and/or diagnostic procedure.

The intra operation data preferably comprises image data and/or scanning data (e.g. ultrasound scanning data) obtained during the minimally invasive surgery and/or diagnostic procedure.

The computer system may advantageously being configured for calculating the volume and/or surface area of a selected part of the body cavity and/or of a selected organ bordering or at least partly within the body cavity. The computer system may e.g. be programmed to determine the volume of a section of an organ within an illuminated hernie, a section of a liver and/or a section of a pouch e.g. in connection with the performing of a gastric bypass operation.

In an embodiment the computer system is configured for receiving instruction representing a periphery of an organ section to be volume determined and to perform the volume determination. The instruction representing the periphery of an organ section may e.g. be provided optically by directing light to mark the periphery of an organ section. In an embodiment the instruction representing the periphery of an organ section is provided digitally via a user interface. Advantageously the computer system being configured for transmitting the calculated data to a presentation device such as a screen and/or a printer and/or other presentation device, such as a tablet, googles or a holographic display.

In an embodiment the computer system is configured for tracking an instrument within the body cavity. The computer system may e.g. be configured for tracking any instrument or a selected instrument within a selected space of the body cavity, such as any instrument or a selected instrument within a space of the body cavity encased by the periphery of the light pattern projected by the optical transmitter device.

Advantageously the computer system preferably is configured for real time tracking of one or more instruments within a selected space.

In an embodiment the computer system is configured for transmitting the calculated data to a screen, wherein the calculated data comprises data representing an argumented reality view.

Advantageously the computer system is operatively connected to the optical transmitter device for operating the optical transmitter device. The computer system may be configured to operate the optical transmitter device at least partly based on the calculated data. For example the computer system may be configured to operate the optical transmitter device based on the calculated data and a pre-programmed algorithm, look-up data, input data via a user interface, input data from one or more additional sensors or any combinations thereof.

Advantageously the computer system is operatively connected to the associated detector device for operating the associated detector. The computer system is for example operatively connected to the associated detector device for operating the associated detector at least partly based on the calculated data, such as based on the calculated data and a pre-programmed algorithm, look-up data, input data via a user interface, input data from one or more additional sensors or any combinations thereof.

In an embodiment computer system is further operatively connected to a surgical intervention instrument for operating the surgical intervention instrument, such as a laparoscopic instrument. The computer system is advantageously configured to operate the surgical intervention instrument at least partly based on the calculated data, and preferably based on the calculated data and a pre-programmed algorithm, look-up data, input data via a user interface, input data from one or more additional sensors or any combinations thereof.

In an embodiment, the computer system is configured for stitching together data sets, such as sets of data representing a topological determination of a tissue field. In an embodiment the computer system is configured for performing topological stitching comprising stitching data sets representing at least two topological determinations, such as from 3 to 100 of the topological determinations, such as from 5 to 50 of the topological determinations.

The topological determination may e.g. comprise determining a plurality of points of the tissue field in 3D for example comprising the spatially relation between the points to obtain a point cloud and the topological stitching may comprise stitching point clouds of said topological determinations to a super point cloud comprising the point clouds spatially combined with each other to represent a larger and/or refined topological determination of the tissue field. The computer system may be configured to perform further 3D determinations, such as volume determinations from the super point cloud.

All features of the inventions and embodiments of the invention as described herein including ranges and preferred ranges may be combined in various ways within the scope of the invention, unless there are specific reasons not to combine such features.

## Brief description of preferred embodiments and elements of the invention

The above and/or additional objects, features and advantages of the present invention will be further elucidated by the following illustrative and nonlimiting description of embodiments of the present invention, with reference to the appended drawings.

The figures are schematic and are not drawn to scale and may be simplified for clarity. Throughout, the same reference numerals are used for identical or corresponding parts.

Fig. 1 is a perspective side view of an embodiment of the optical transmitter device of the invention.

Fig. 2 is a schematic illustration of another embodiment of the optical transmitter device of the invention.

Figs. 2a-2d illustrates different examples of cross sectional shapes of the optical transmitter device e.g. as seen in the cross sectional cut A-A of Fig. 2.

Fig. 3 is schematic illustration of a variation of the embodiment of Fig. 2

Figs. 4a-4c illustrates a cross-sectional view of a part of an embodiment of the optical transmitter device of the invention, wherein the optical transmitter device comprises an intra cavity protection arrangement and the projector is arranged in a channel of the distal portion of the penetrator member when the penetrator tip is in its penetrating position.

Fig. 5 is a schematic illustration of an embodiment of the optical transmitter device wherein the distal portion of the penetrator member comprises a curvilinear length section.

Figs. 6a - 6c illustrates a cross-sectional view of a distal portion of an embodiment of the penetrator member, wherein the penetrator member comprises a sleeve and the projector is incorporated in the sleeve.

Fig. 7 is a schematic illustration of the distal portion of the penetrator member of an embodiment of the optical transmitter device wherein the projector is incorporated in the penetrator tip.

Figs. 8a-8c illustrates a cross-sectional view of a part of an embodiment of the optical transmitter device of the invention, wherein the optical transmitter device comprises an intra cavity protection arrangement and the optical transmitter device comprises two projector projectors which are arranged in non-central channels of the distal portion of the penetrator member.

Figs. 9a and 9b illustrates a part of an embodiment of the penetrator member, wherein the projector has a first folded position and a second unfolded/pivoted position.

Figs. 10a-10e illustrates examples of shapes of suitable penetrator tips for embodiments of the optical transmitter device.

Fig. 11 is a schematic illustration of an embodiment of a 3D sensor system comprising an optical transmitter device and an associated detector, wherein the 3D sensor system is illustrated in a cross-sectional view of a body cavity when the 3D sensor system is in use for diagnostic and/or surgical purpose.

Fig. 12 is a schematic illustration of another embodiment of a 3D sensor system, wherein the comprising an optical transmitter device, wherein the 3D sensor system comprises a computer system.

Figs. 13a - 13f illustrate different examples of projected patterns.

Fig. 14a is a schematic illustration seen in a cross-sectional view of a part of an associated structured lightning member. The associated structured lightning member comprises a not shown light source and a waveguide - optical projector assembly suitable for forming part of an embodiment of the optical transmitter device, wherein the optical projector comprises an optical filter.

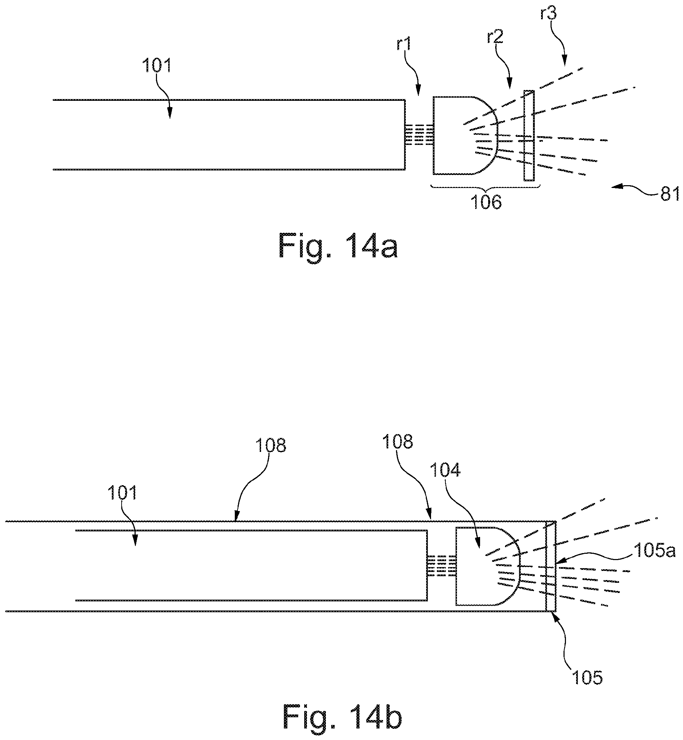

Fig. 14a is show the waveguide -optical projector assembly in a cross-sectional view and enclosed in a hermetic housing.

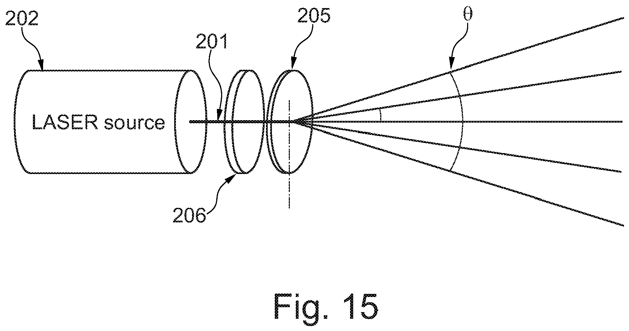

Fig. 15 is a schematic illustration of an associated structured lightning member comprising a light source - waveguide -optical projector and focusing lens assembly suitable for forming part of an embodiment of the optical transmitter device.

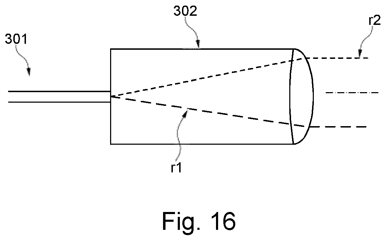

Fig. 16 is a schematic illustration of a beam expanding lens arrangement suitable for an associated structured lightning member.

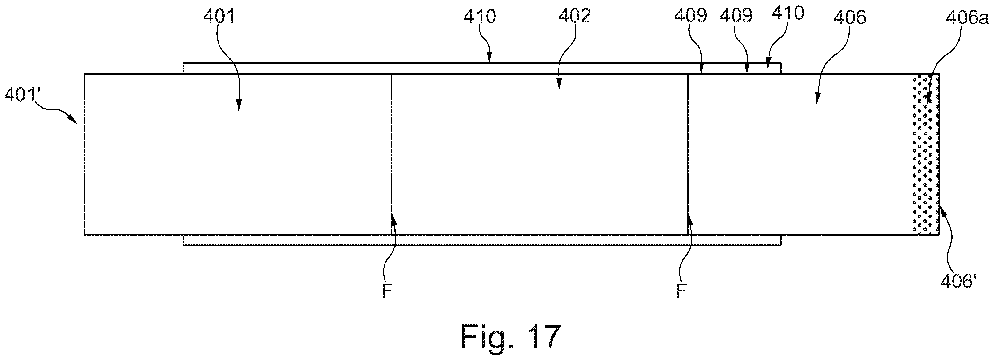

Fig. 17 is a schematic illustration of an embodiment of a projector probe of the invention.

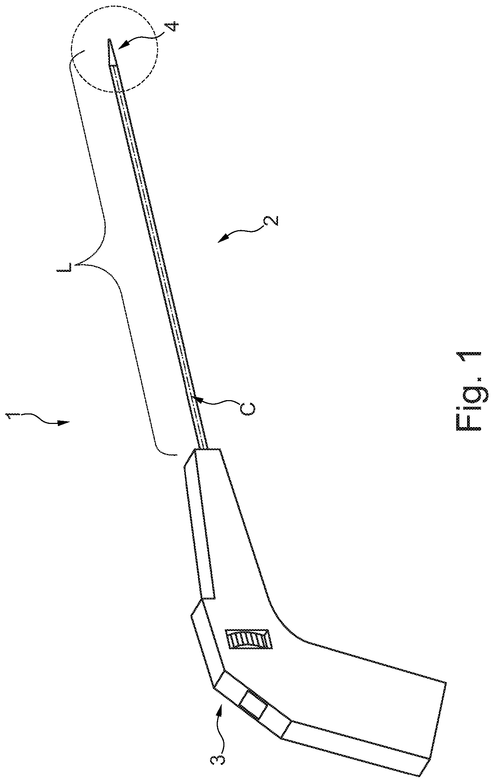

Further scope of applicability of the present invention will become apparent from the description given hereinafter. However, it should be understood that the detailed description and specific examples, while indicating preferred embodiments of the invention, are given by way of illustration only, since various changes and modifications within the scope of the claimed invention will become apparent to those skilled in the art from this detailed description. The optical transmitter device shown in Fig 1 comprises an elongate penetrator member 1 suitable for penetrating through mammal skin. The penetrator member has a distal portion 2 and a proximal portion 3. The proximal portion 3 comprises in this embodiment a handle and thus the optical transmitter device is suitable for manual handling. The distal portion has a center axis C and a length L and comprises a penetrator tip 4. The optical transmitter device further comprises a not shown associated structured lightning member comprising a light source and a projector, wherein the projector disposed at the distal portion 2 of the penetrator member and is configured for projecting a diverging light pattern.

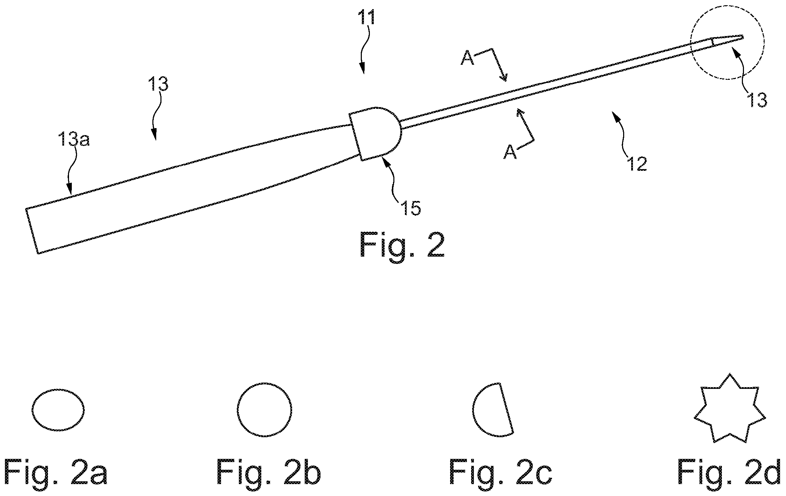

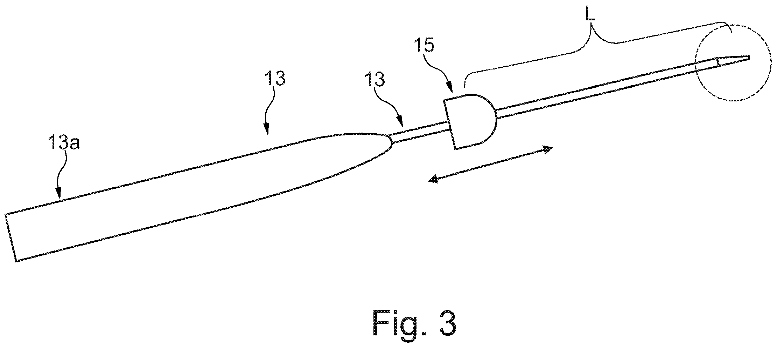

The optical transmitter device shown in Fig. 2 comprises an elongate penetrator member 11 suitable for penetrating through mammal skin. The penetrator member has a distal portion 12 and a proximal portion 13. The proximal portion 13 comprises in this embodiment a connector 13a for being connected to or integrated with a robot for robotic operation. The penetrator member 11 further comprises an obstruction 15 arranged to define a boundary between the distal portion 12 and the proximal portion 13 of the penetrator member 11 and for preventing intrusion of the proximal portion into the skin. As further shown in Fig. 3 the obstruction 15 may preferable be displaceable.

The distal portion has a center axis and a length and comprises a penetrator tip 14. The optical transmitter device further comprises an associated structured lightning member comprising a projector disposed at the distal portion 2 of the penetrator member.

As mentioned above the distal portion of the penetrator member may have any cross-sectional shape, however generally it is desired that except for the penetrator tip the penetrator member does not have sharp edges which unintentionally may damage the skin of the patient.

Figs. 2a-2d illustrates different examples of cross sectional shapes of the optical transmitter device e.g. as seen in the cross sectional cut A-A of Fig. 2.

Fig. 2a illustrates an example where the distal portion of the penetrator member has an oval cross-sectional periphery. Fig. 2b illustrates an example where the distal portion of the penetrator member has a substantially circular cross-sectional periphery. Fig. 2c illustrates an example where the distal portion of the penetrator member has a semi-circle cross-sectional periphery. Fig. 2d illustrates an example where the distal portion of the penetrator member has a star shaped cross-sectional periphery.

Fig. 3 is schematic illustration of a variation of the embodiment of Fig. 2 wherein the obstruction 15 is displaceable such that the surgeon may preselect the length of the distal portion of the penetrator member.

Thereby the length of the distal portion of the penetrator member is selectable and the obstruction 15 may be positioned to ensure that the length of the distal portion of the penetrator member is adapted to the size and depth of the body cavity and to the thickness of the skin to be penetrated such that the risk of penetrating deeper than desired and e.g. unduly damage tissue within the cavity may be prevented. The obstruction 15 may additionally be used to determine the penetration depth of the distal portion of the penetrator member.

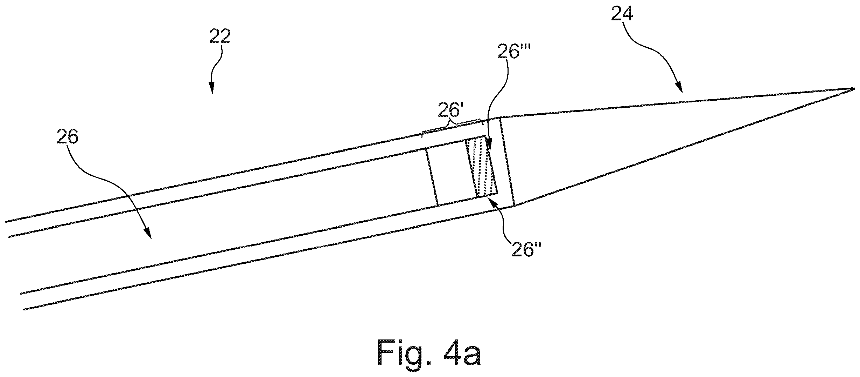

Figs. 4a-4c illustrates a distal part of an embodiment of the optical transmitter device comprising the penetrator tip. The distal part of the optical transmitter device may e.g. be or correspond to the part encircled by a dotted line in respectively Figs. 1, 2 or 3 and it may comprise at least a part of the distal portion of the penetrator member including the penetrator tip.

The illustrated distal portion 22 of the penetrator member comprises a lumen and the projector 26' in the form of a part of a projector probe 26 is disposed in the lumen. The lumen forms part of a channel extending in and along the length of the distal portion of the penetrator member. Thereby the projector may be protected by the penetrator member during the introduction through the skin and into the body cavity. In the shown embodiment the channel including the lumen is substantially centrally positioned relative to the axis of the distal portion 22 of the penetrator member.

The projector may advantageously comprise a front layer 26"comprising elements or ions which are absorbing light of at least one wavelength of the light source while it is allowing substantially all light of at least one other wavelength of the light source to pass substantially un-attenuated. The front layer with the distal front face 26‴ may e.g. be a separate optical filter or e.g. an optical filter fused to the pattern generating member comprising the DOE.

The optical transmitter device illustrated in Figs. 4a-4c comprises an intra cavity protecting arrangement for protecting against undesired impacts by the penetrator tip after it has been introduced into the body cavity and at the same time the projector or the entire projector probe is protected by the penetrator tip prior to penetrating the skin. The intra cavity protecting arrangement is provided by the structure of the tip 24 and the projector probe 26 which is displaceable within the channel of the distal portion 22 of the penetrator member.

In Fig. 4a the penetrator tip is in its first penetrator position where the penetrator tip is adapted for penetrating the skin into the body cavity. The tip is 24 is sharp for easy penetrating of the skin and the projector probe 26 including the projector 26' is protected within the lumen (closed channel) of the distal portion 22 of the penetrator member. The penetrator tip 24 comprises a number of leaf-like flanges 24a projecting to form the penetrator tip.

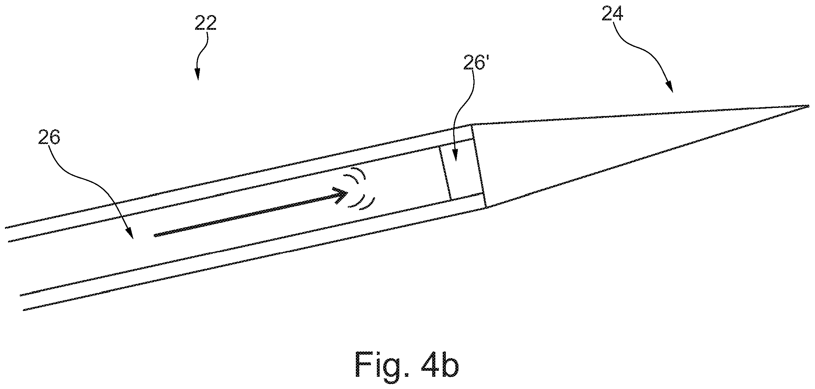

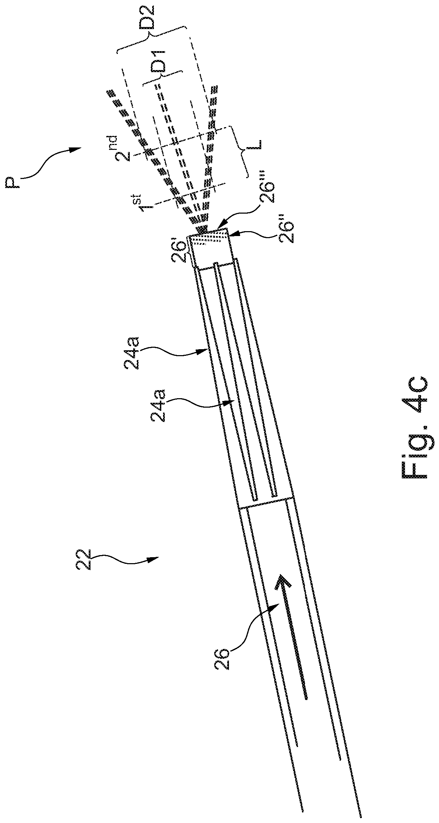

In Fig. 4b the projector probe is being axially displaced in distal direction as indicated with the arrow. In Fig. 4c the projector probe has been fully axially displaced in distal direction to thereby switch the penetrator tip 24 to its second penetrator tip protected position and at the same time the projector 26' is displaced along the axis of the distal portion 22 of the penetrator member into its operating position where the light pattern P is projected from the projector 26' via the optical filter 26". As it can be seen the penetrator tip 24 is now in its protected position by spreading of the tip portions of leaf-like flanges 24a by the projector probe 26.

The optical filter 26" absorbs a certain amount of light which is converted to heat which prevent and/or removes fog/mist on the distal front face 26‴ of the projector 26'.

It can be seen that the pattern P is spreading out from the projector 26', i.e. the projector is projecting a diverging light pattern with a relatively high diverging angle after having introduced the distal portion of the penetrator member sufficiently into the body cavity. The projector may for example be fixed to be pivotally folded from a first covered position to a second projecting (operating) position and/or the projector may be fixed to be axially displaced from a first retracted position to a second projecting position ready for projecting the light pattern within the body cavity e.g. towards a target surface area within the body cavity.

The beam divergence, Θ may be determined as follows:

$$\Theta = \left(2arctan\left(\frac{\frac{1}{2}D2 - \frac{1}{2}D1}{2L}\right)\right.$$

wherein D1 is the largest cross-sectional dimension orthogonal to the center axis of the light pattern a first distance from the projector, D2 is the largest cross-sectional dimension orthogonal to the center axis of the light pattern a second larger distance from the projector and L is the distance between D1 and D2 and wherein the distances is determined along the center axis of the light pattern.

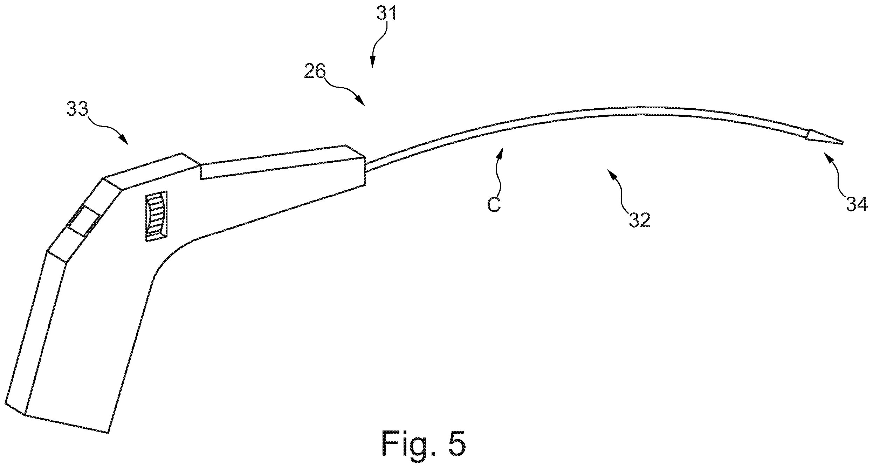

The optical transmitter device shown in Fig 5 comprises an elongate penetrator member 31 suitable for penetrating through mammal skin. The penetrator member has a distal portion 32 and a proximal portion 3. The proximal portion 33 comprises in this embodiment a handle and thus the optical transmitter device is suitable for manual handling. It should be understood that the handle could be replaced by a connector for being connected to or integrated with a robot for robotic operation of the optical transmitter device.

The distal portion 32 of the penetrator member comprises a curvilinear length section 32'. The curving of the distal portion of the penetrator member may for some application make it simpler for a surgeon to insert the penetrator tip and the not shown projector into a body cavity. For example where the body cavity is partly enclosed by bone material or organ(s) which are desirably not penetrated it may be advantageous that the distal portion of the penetrator member is fully or partly curved.

In an embodiment the curvilinearity of the curvilinear length section may be selectable by the surgeon e.g. by providing at least the curvilinear length section in a rigid but plastic deformable material. The surgeon may thus adjust the distal portion of the penetrator member to have a desired curvilinearity prior to penetrating the skin of the mammal.

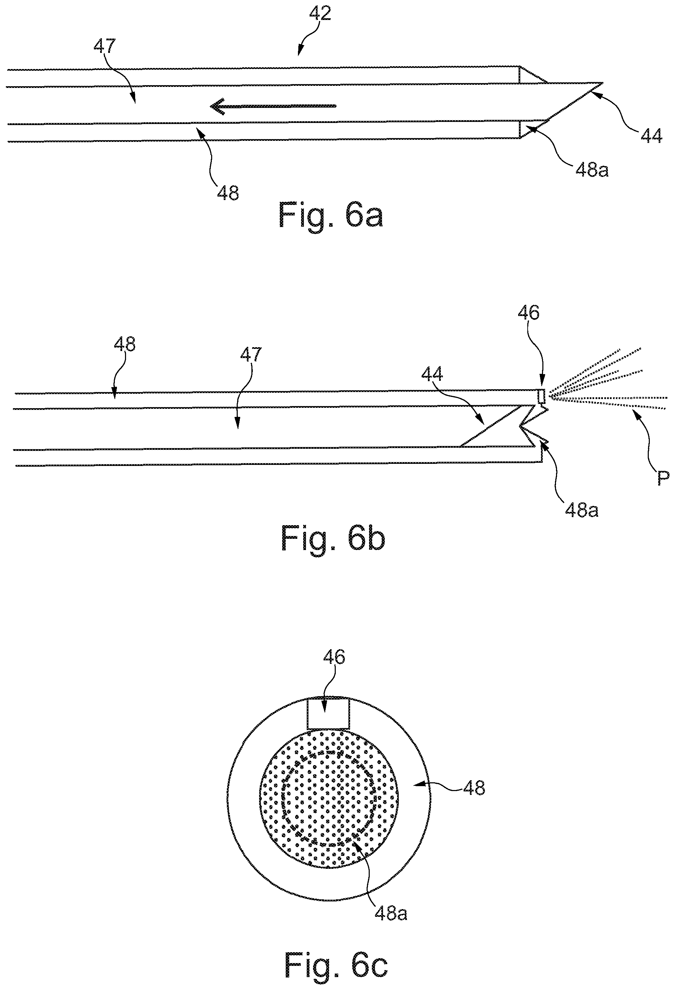

The optical transmitter device illustrated in Figs. 6a - 6c comprises an intra cavity protecting arrangement for protecting against undesired impacts by the penetrator tip after it has been introduced into the body cavity optionally also prior to being penetrated into the body cavity. Simultaneously the projector is protected by the sleeve during the penetrating through the skin.

The optical transmitter device illustrated in Figs. 6a - 6c comprises a distal portion 42 of the penetrator member comprising a penetrator body 47 surrounded by a sleeve 48. At the distal end of the penetrator body 47 it comprises a penetrator tip 44 having a bevel tip shape. At the distal end of the sleeve 48 it comprises a sleeve flange 48a. A light guide - preferably an optical fiber and a projector 46 is incorporated /protected by the sleeve 48 and the sleeve flange 48a.

In Fig. 6a the penetrator tip is in its first penetrator position where the penetrator tip 44 is adapted for penetrating the skin into the body cavity. The tip is 44 is sharp for easy penetrating of the skin and the sleeve flange 48a forms a beveled support face for optimizing penetration. At the same time the sleeve flange 48a protects the projector 46 including the distal front face of the projector.

As indicated by the arrow the penetrator can be axially displaced in proximal direction to thereby switch the penetrator tip 44 to its second penetrator tip protected position.

In Fig. 6b the optical transmitter device is illustrated with its penetrator tip 44 in its second penetrator tip protected position. As it can be seen the sleeve flange 48a is folded inwards to cover the penetrator tip 44 and at the same time at least the distal front face of the projector 46 is exposed for projecting a light pattern P within the body cavity.

Fig. 6c is a front view of the optical transmitter device with its penetrator tip 44 in its second penetrator tip protected position. It can be seen that the penetrator tip 44 is fully covered by the sleeve flange 48a.

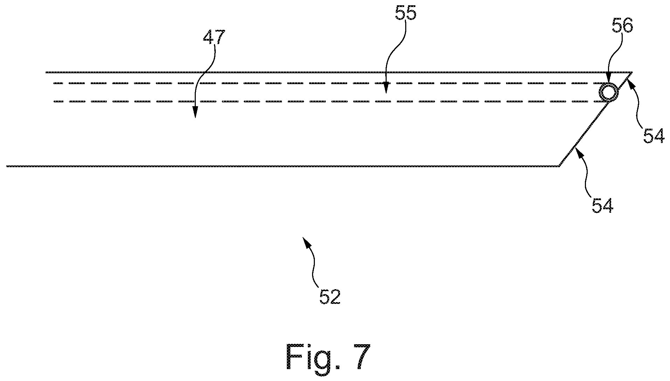

The optical transmitter device with the distal portion 52 of the penetrator member illustrated in Fig. 7 is of a relatively simple type and comprises a penetrator body 57 with a distal penetrator tip 54. A projector 56 for projecting the light pattern is arranged at the penetrator tip 54. A channel 55, illustrated by dotted lines, comprises a not shown waveguide - e.g. an optical fiber which is arranged for guiding light from a not shown light source to the projector 56.

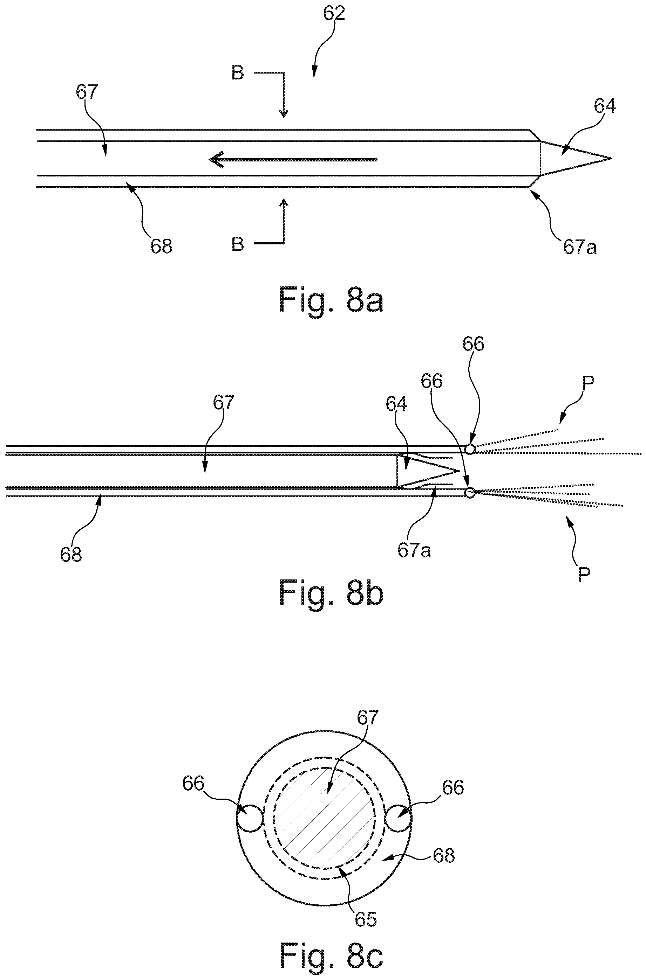

The optical transmitter device illustrated in Figs. 8a-8c comprises an intra cavity protection arrangement and the optical transmitter device comprises two projectors 66, which are arranged in non-central channels of the distal portion 62 of the penetrator member. The intra cavity protecting arrangement is arranged for protecting against undesired impacts by the penetrator tip after it has been introduced into the body cavity optionally also prior to being penetrated into the body cavity. Simultaneously the projectors 66 are protected during the penetrating through the skin.

The optical transmitter device illustrated in Figs. 8a - 8c comprises a distal portion 62 of the penetrator member comprising a penetrator body 67 surrounded by a sleeve 68. At the distal end of the penetrator body 67 it comprises a penetrator tip 64 having a conic tip shape. The penetrator body may comprise or consist of a projector probe as described above. At the root of the penetrator tip 64, a flexible projector cover flange 67a is arranged e.g. made of silicone rubber or of organic polymer. A light guide - preferably an optical fiber is incorporated /protected by the sleeve 68 and is arranged for guiding light to the projectors 66.

In Fig. 8a the penetrator tip 64 is in its first penetrator position where the penetrator tip 64 is adapted for penetrating the skin into the body cavity. The tip is 64 is sharp for easy penetrating of the skin and the projector cover flange 67a forms a beveled support face for optimizing penetration. At the same time the projector cover flange 67a protects the projectors 66 including the distal front face of each of the projectors.

As indicated by the arrow the penetrator can be axially displaced in proximal direction to thereby switch the penetrator tip 64 to its second penetrator tip protected position.

In Fig. 8b the optical transmitter device is illustrated with its penetrator tip 64 in its second penetrator tip protected position. As it can be seen the projector cover flange 67a is retracted together with the penetrator tip 64 into the channel 65 encircled by the sleeve 68 at least the distal front face of the respective projectors 66 are exposed for projecting respective light patterns P within the body cavity. The sleeve 68 protects the penetrator tip 64 for unintended penetration of tissue within the body cavity.

Fig. 8c is a cross-sectional view of the distal portion 62 of the penetrator member seen in the cut B-B. It can be seen that the penetrator body 67 is arranged in the channel 65 encircled by the sleeve 68 and the two channels 66' for the waveguides and the projectors 66 are arranged radially outwards relative to the axis of the distal portion 62 of the penetrator member. The two channels 66' for the waveguides and the projectors 66 are for example arrange at respectively 3 o'clock and a 9 o'clock using the 12-hour clock system.

The optical transmitter device illustrated in Figs. 8a-8c comprises an intra cavity protection arrangement and the optical transmitter device comprises two projectors 66, which are arranged in non-central channels of the distal portion 62 of the penetrator member. The intra cavity protecting arrangement is arranged for protecting against undesired impacts by the penetrator tip after it has been introduced into the body cavity optionally also prior to being penetrated into the body cavity. Simultaneously the projectors 66 are protected during the penetration through the skin.

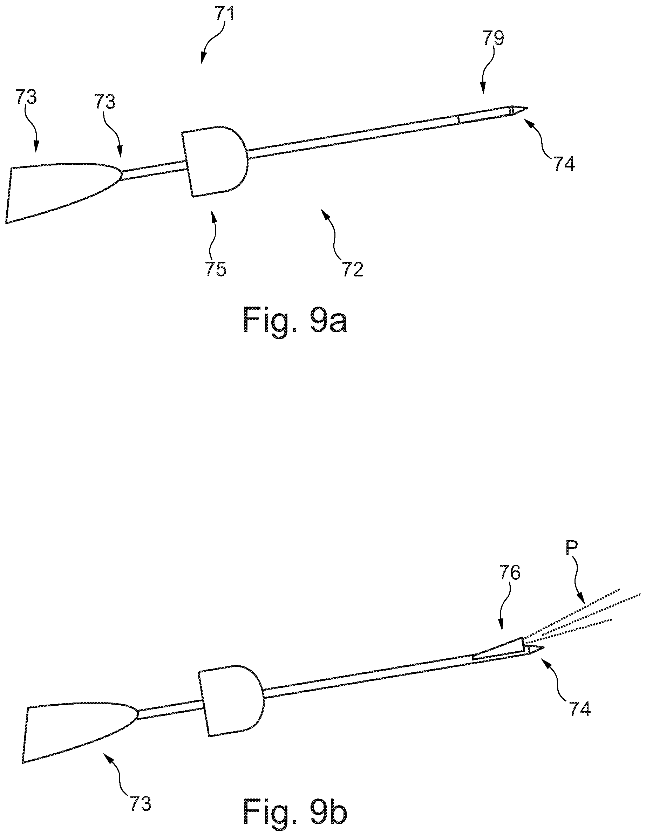

In the embodiment of the optical transmitter device shown in Figs. 9a and 9b it comprises a penetrator member 71 with a proximal portion 73 and a distal portion 72. The penetrator member 71 further comprises an obstruction 75 arranged to define a boundary between the distal portion 72 and the proximal portion 73 of the penetrator member 71 and for preventing intrusion of the proximal portion into the skin. The distal portion 72 comprises a distally arranged penetrator tip 74. The distal portion 72 of the penetrator member further comprises a lumen which position is illustrated with the ref. 79. The optical transmitter device further comprises an associated structured lightning member comprising a projector 76 disposed at the distal portion 72 of the penetrator member a not shown light source operationally connected to the projector 76.

In Fig. 9a the optical transmitter device is shown with the projector 76 in a first covered position wherein the projector is folded into the lumen 79 of the distal portion 72 of the penetrator member. In this position the projector 76 does not protrude axially outwards from the distal portion 72 of the penetrator member. Further the projector may be protected against dust or other undesired contaminations when the optical transmitter device is outside the body cavity.

In Fig. 9b the optical transmitter device is shown with the projector 76 in its second projecting (and operating) position where the projector is unfolded or pivoted. When the distal portion of the penetrator member is retracted from the body cavity the projector 76 will automatically be folded back to its first covered position.

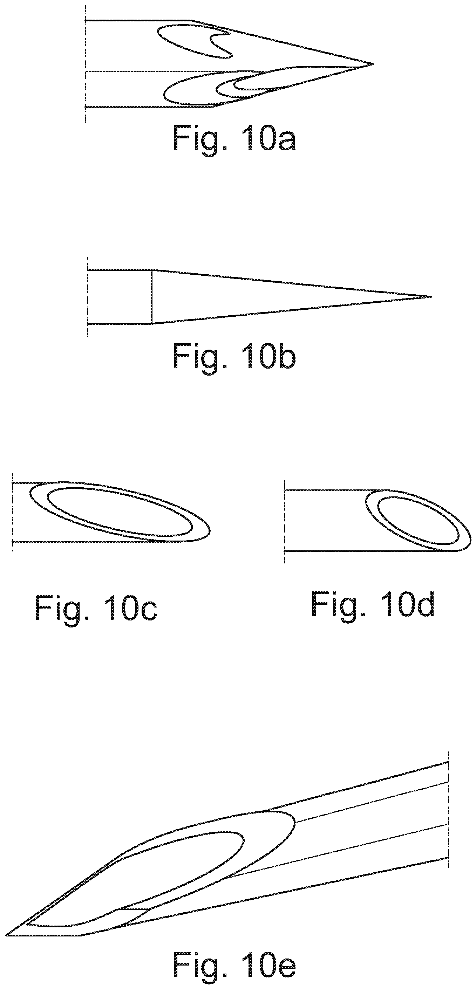

Figs. 10a-10e illustrates examples of shapes of suitable penetrator tips for embodiments of the optical transmitter device. Fig. 10a illustrates a triangularly (Franseen) shaped penetrator tip. Fig. 10b illustrates a conically shaped penetrator tip. Fig. 10c illustrates a beveled shaped penetrator tip with a bevel angle of about 22 degrees. Fig. 10d illustrates a beveled shaped penetrator tip with a bevel angle of about 45 degrees. Fig. 10e illustrates a beveled shaped penetrator tip with a bevel angle of about 18 degrees.

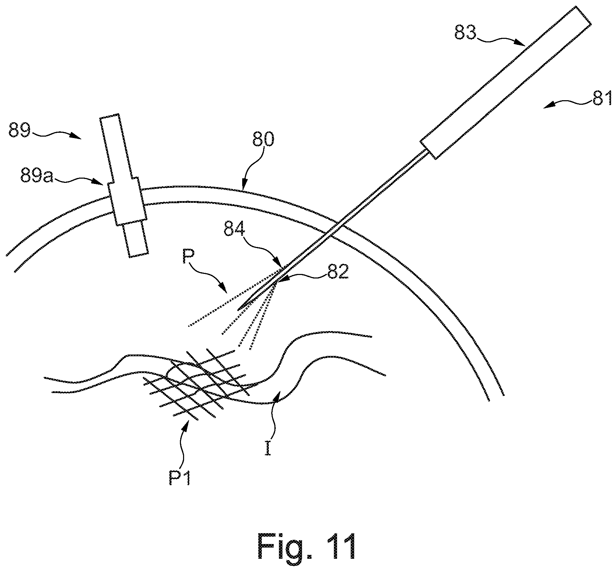

Fig. 11 illustrates a cross sectional view into a body cavity 85 e.g. an abdominal body cavity. A 3D sensor system 83, 89 is in use for performing a diagnostic procedure and/or a surgical procedure. The 3D sensor system comprises a camera 89 associated to an optical transmitter device 81. The camera 89 has a distal camera portion introduced via a cannula 89a into the body cavity 85. The cannula 89a is inserted through an incision into the skin 80 of the mammal e.g. using a trocar. The 3D sensor system further comprises the optical transmitter device 81 e.g. as described above and comprising a proximal portion 83 and a distal portion 82 with a penetrator tip 84 and a projector introduced into the body cavity 85. The distal portion 82 of the penetrator member can be introduced into the body cavity 85 simply by penetrating the skin 80 using the penetrator tip 84 i.e. without any precutting needs to be provided into the skin. Thus, the injury caused by the optical transmitter device 81 is very little and after the optical transmitter device 81 has been removed, the penetrating hole in the skin may close by itself without requiring suture.

The projector projects a light pattern P towards a target surface area e.g. an intestine I. The reflected pattern P1 is recorded by the associated camera 89 and transmitted to a not shown computer system of the 3D sensor system. The computer system is programmed to calculating data representing a 3D surface contour of a surface area reflecting light transmitted from the optical transmitter device based on 3D signal data from the optical transmitter device and detected data from the detector.

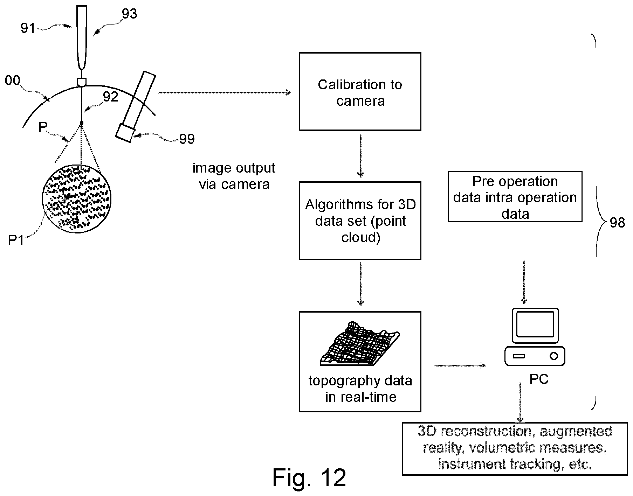

The 3D sensor system 3D sensor system illustrated in Fig. 12 comprises a optical transmitter device 91 e.g. as described above, an associated detector 99 e.g. in the form of a camera and a computer system 98.

The optical transmitter device 91 comprises a proximal portion 93 and a distal portion 92 with a penetrator tip and a projector introduced into the body cavity via the skin 90. The projector projects a light pattern P towards a target surface area. The reflected pattern P1 is recorded by the associated camera 99 and transmitted to the computer system 98. The computer system is configured for calibration of the camera and comprises algorithms for calculating a point cloud representing the 3D contour of the target area. Thus the computer system may generate topography data in real time. The computer system is advantageously also in data communication with a sensor or a robot for receiving position date of the projector and preferably also for receiving angular position data representing the angle between the optical axis of the projector and the optical axis of the associated detector of the 3D sensor system.

The computer is advantageously further in data communication with or comprises a database for receiving pre operation data and for using such pre operation data in its calculation.

The pre operation data is for example data from a CT scan or a MR scan or data estimated based on age, gender and/or weight of the patient or as further described above.

Based on the collected data the computer system is configured for calculating a 3D reconstruction data of a least a target area.

In an embodiment the computer system is configured for receiving intra operation data representing at least one attribute of the body cavity during the minimally invasive surgery and/or diagnostic procedure and for using said intra operation data for calculating data representing a 3D reconstruction of at least a part of a body cavity. The intra operation data may be as described above, such a data from a CT scanning and/or from a MR scanning.

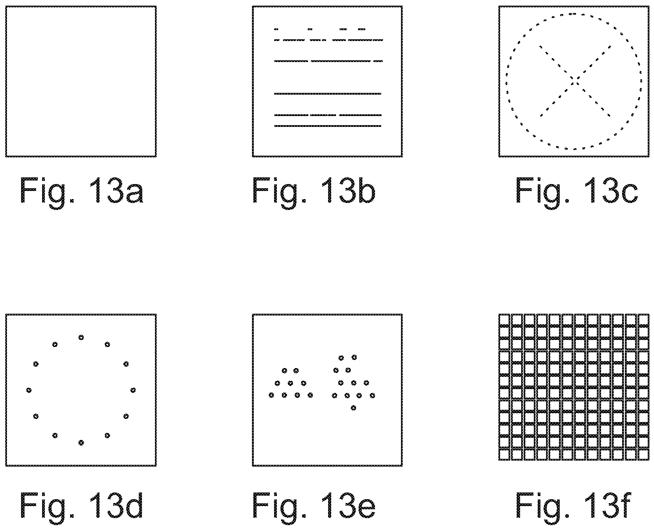

The computer system advantageously further calculate augumented reality data and/or volumetric data and/or instrument tracking data e.g. as described above. Figs. 13a - 13f illustrate different examples of projected patterns. The skilled will understand the pattern may have many other shapes than the illustrated e.g. symmetrical shapes, asymmetrical shapes and e.g. as described above optionally including a combination of one or more of the before mentioned shapes.

The associated structured lightning member illustrated in Fig. 14a comprises a not shown light source, a waveguide 101 and an optical projector 106, wherein the waveguide 101 and the optical projector 106 forms an assembly. The waveguide 101 is illustrated as an optical fiber. The projector 106 comprises a DOE 104 and an optical filter 105. The optical filter may e.g. be as described above. In an embodiment the optical filter is a SCHOTT glass optical filter available from SCHOTT Advanced Optics.

As illustrated the light L1 emitted from the optical fiber 101 is emitted with essentially parallel rays r1. The DOE disperse the rays r2 to have the desired a divergent angle. The optical filter absorbs at least one wavelength of the optical source and allows the remaining light rays r3 to pass substantially un-attenuated. The absorbed light is transformed to light and the optical filter is heated to prevent and/or remove fog/mist from the distal front face 105a.

In fig. 14 the waveguide -optical projector assembly 101, 106 is enclosed in a hermetic housing 108 ensuring that fig or mist is not formed or accumulated between the respective elements. In a variation thereof the various elements, the optical fiber 101, the DOE 104 and the optical filter is fused together.

The associated structured lightning member shown in Fig. 15 comprises a light source 202, a waveguide 201, an optical projector 206 and focusing lens 205. The waveguide 201 is an optical fiber and the optical projector 206 is a DOE. The light pattern is projected in the desired direction and focused by the focusing lens 205. The projected pattern has a diverging angle Θ.

The beam expanding lens arrangement illustrated in Fig. 16 comprises a beam expanding lens 302 having a length L. As illustrated the light is fed from a not shown light source via an optical fiber 301 to the proximal end of the beam expanding lens 302. The light enters the beam expanding lens and due to a continuous change of the refractive index within the lens material the light rays r1 are continuously bent to thereby expand the diameter of the beam as the light propagates through the beam expanding lens 302 along its length L. At the exit of the beam expanding lens 302 the light is collimated to form a beam with substantially parallel rays r2 of light. Hereafter the collimated light may be transmitted further to the DOE.

The projector probe of Fig. 17 comprises an optical fiber 401 with a proximal end 401'and a distal end, a beam expanding lens 402 and a projector 406 with a distal front face 406'. The optical fiber 401, the beam expanding lens 402 and the projector 406 is fused in the fused interfaces F. The optical fiber 401, the beam expanding lens 402 and the projector 406 is arranged in a hermetic metal housing 410 preferably using epoxy seal 409. When a light beam is pumped from a not shown light source into the proximal end 401' of the optical fiber 401 the light will propagate through the optical fiber 401 collimated in the core of the optical fiber. From the fiber 401 it will pass into the beam expanding lens 402 which is advantageously a GRIN lens and the beam will expand as the light propagates through the beam expanding lens 402. At the exit of the beam expanding lens 402 the light will be collimated and it will propagate into the projector which is advantageously a DOE. Here the light patter will be shaped the projector will project a divergent light pattern. The projector may advantageously comprise an optical filter or an optical filter layer as described above to prevent and/or remove fog/mist. The optional optical filter or an optical filter layer is indicated with the dotted part 406a of the projector 406.

**Claims**

1. An optical transmitter device for emitting light within a body cavity, said optical transmitter device (81, 91) comprises an elongate penetrator member (1, 11, 31, 71) for penetrating through mammal skin and an associated structured lightning member, the penetrator member (1, 11, 31, 71) has a distal portion (2, 12, 22, 32, 42, 52, 62, 72, 82, 92) and a proximal portion (3, 33, 73, 83, 93), wherein the distal portion (2, 12, 22, 32, 42, 52, 62, 72, 82, 92) has a center axis and a length (L) and comprises a penetrator tip (4, 14, 24, 34, 44, 54, 64, 74, 84), the structured lightning member comprises a light source and a projector (26', 46, 56, 66, 76, 106, 206, 406), wherein the light source is operatively connected to the projector (26', 46, 56, 66, 76, 106, 206, 406) by a light guiding structure (201, 301, 401), the light guiding structure and the projector form part of a projector probe, the projector (26', 46, 56, 66, 76, 106, 206, 406) is disposed at the distal portion (2, 12, 22, 32, 42, 52, 62, 72, 82, 92) of the penetrator member (1, 11, 31, 71) and is configured for projecting a diverging light pattern and wherein the projector comprises a diffractive optical element (DOE) (104) comprising a microstructured surface configured for creating the light pattern and further comprises a beam expanding lens arrangement comprising a gradient index (GRIN) lens (402) arranged prior to the DOE (104, 406), preferably the projector (26', 46, 56, 66, 76, 106, 206, 406) has at least one position wherein it is configured for projecting the light pattern outwards relative to the optical transmitter device, preferably for projecting the light pattern distally relative to the optical transmitter device.

2. The optical transmitter device of claim 1, wherein said distal portion (2, 12, 22, 32, 42, 52, 62, 72, 82, 92) of the penetrator member has a length (L) of at least about 0.5 cm and a maximal cross-sectional dimension of up to about 1 cm, such as up to about 2 mm.

3. The optical transmitter device of any one of the preceding claims, wherein said light guiding structure comprising an optical fiber (201, 301, 401) having a light output end configured for transmitting an output light beam towards the diffractive optical element (104, 406).

4. The optical transmitter device of claim 3, wherein said projector probe comprises said gradient index (GRIN) lens (402) between the optical fiber light output end and said DOE (406).

5. The optical transmitter device of any one of claim 3 or claim 4, wherein said light guiding structure has a beam output with a beam output spot diameter of at

least about 4 $\mu$m and wherein said light source is operatively connected to the projector (26', 46, 56, 66, 76, 106, 206, 406) such that a beam of light impinges onto the projector, said impinging beam of light has a beam diameter of at least about 0.4 mm, such as at least about 0.6 mm, such as at least about 0.7 mm.

6. The optical transmitter device of any one of the preceding claims,
   wherein said optical transmitter device comprises a tip protecting arrangement configured for fully or partly covering the tip (64) after the distal portion (62) of the penetrator member has penetrated the skin and entered into a body cavity, said tip protecting arrangement preferably comprises a flange (67a) optionally forming part of the projector and/or the projector probe.

7. The optical transmitter device of any one of the preceding claims,
   wherein said optical transmitter device comprises an intra cavity protecting arrangement for protecting against undesired impacts by the penetrator tip within a body cavity, preferably the intra cavity protecting arrangement is configured for switching the penetrator tip between a first penetrator position and a second penetrator tip protected position.

8. The optical transmitter device of any one of the preceding claims,
   wherein said projector (26', 46, 56, 66, 76, 106, 206, 406) is configured for projecting the light pattern with a diverging angle of at least about 10 degrees, such as at least about 20 degrees, such as at least about 30 degrees, such as at least about 40 degrees relative to the optical axis of the light pattern, preferably the diverging angle is selectable.

9. The optical transmitter device of any one of the preceding claims,
   wherein said light pattern comprises structured light comprising areas of light and areas of no-light and/or areas of light of a first quality of a character and areas of light of a second quality of the character, wherein the character advantageously is selected from light intensity, wavelength and/or range of wavelengths.

10. A 3D sensor system comprising an optical transmitter device according to any one of the preceding claims and an associated detector (99), wherein said associated detector (99) is configured for detecting reflected rays of the light pattern projected by the projector of the optical transmitter device.

11. The 3D sensor system of claim 10, wherein said 3D sensor system comprises a computer system (98), said computer system being operatively connected to said optical transmitter device (81, 91) and said associated detector (99) and being configured for calculating data representing a 3D surface contour of a surface area reflecting light transmitted from said optical transmitter device based on 3D signal data from said optical transmitter device and detected data from said associated detector.

12. The 3D sensor system of claim 11, wherein said computer system (98) is configured for receiving angular position data representing the angle between the optical axis of the projector and the optical axis of the associated detector (99) and/or the computer system is configured for receiving data for calculating said angular position data.

13. The 3D sensor system of any one of claims 10-12, wherein said 3D sensor system forms part of an operator in the form of a robot, said transmitter device (81, 91) and said associated detector being connected to or being integrated with said robot and said 3D sensor system being configured for real time operation, said computer system being configured for real time calculating of data representing said 3D surface contour representing a 3D reconstruction of at least a part of a body cavity.

14. The 3D sensor system of any one of claims 11-13, wherein said computer system (98) being configured for receiving pre operation data and/or intra operation data representing at least one attribute of the body cavity and for using said pre operation data for calculating data representing a 3D reconstruction of at least a part of a body cavity, said pre operation data preferably comprises image data and/or scanning data and/or corresponding estimated data, calculated data and/or scanning data obtained during the minimally invasive surgery and/or diagnostic procedure.

**Patentansprüche**

1. Optische Sendervorrichtung zum Emittieren von Licht in einer Körperhöhle, wobei die optische Sendervorrichtung (81, 91) ein längliches Durchdringungselement (1, 11, 31, 71) zum Durchdringen von Säugetierhaut und ein zugehöriges strukturiertes Blitzelement umfasst, wobei das Durchdringungselement (1, 11, 31, 71) einen distalen Abschnitt (2, 12, 22, 32, 42, 52, 62, 72, 82, 92) und einen proximalen Abschnitt (3, 33, 73, 83, 93) aufweist, wobei der distale Abschnitt (2, 12, 22, 32, 42, 52, 62, 72, 82, 92) eine Mittelachse und eine Länge (L) aufweist und eine Durchdringungsspitze (4, 14, 24, 34, 44, 54, 64, 74, 84) umfasst, das strukturierte Blitzelement eine Lichtquelle und einen Projektor (26', 46, 56, 66, 76, 106, 206, 406) umfasst, wobei die Lichtquelle mit dem Projektor (26', 46, 56, 66, 76, 106, 206, 406) durch eine Lichtleitstruktur (201, 301, 401) betriebsmäßig verbunden ist, die Lichtleitstruktur und der Projektor einen Teil einer Projektorsonde bilden, der Projektor (26', 46, 56, 66, 76, 106, 206, 406) an dem distalen Abschnitt (2, 12, 22, 32, 42, 52, 62, 72, 82, 92) des Durchdringungselements (1, 11, 31, 71) angeordnet und zum Projizieren eines divergierenden Lichtmusters konfiguriert ist, und wobei der Projektor ein diffraktives optisches Element (DOE) (104) umfasst, das eine mikrostrukturierte Oberfläche umfasst, die zum Erzeugen des Lichtmusters konfiguriert ist, und weiter eine Strahlaufweitungslinsenanordnung umfasst, die eine Gradientenindex- (GRIN) Linse (402) umfasst, die vor dem DOE (104, 406) angeordnet ist, wobei der Projektor (26', 46, 56, 66, 76, 106, 206, 406) vorzugsweise mindestens eine Position aufweist, wobei er zum Projizieren des Lichtmusters nach außen relativ zu der optischen Sendervorrichtung konfiguriert ist, vorzugsweise zum Projizieren des Lichtmusters nach distal relativ zu der optischen Sendervorrichtung.

2. Optische Sendervorrichtung nach Anspruch 1, wobei der distale Abschnitt (2, 12, 22, 32, 42, 52, 62, 72, 82, 92) des Durchdringungselements eine Länge (L) von mindestens etwa 0,5 cm und eine maximale Querschnittsabmessung von bis zu etwa 1 cm, wie etwa bis zu etwa 2 mm, aufweist.

3. Optische Sendervorrichtung nach einem der vorstehenden Ansprüche, wobei die Lichtleitstruktur eine optische Faser (201, 301, 401) mit einem Lichtausgangsende umfasst, das zum Senden eines Ausgangslichtstrahls in Richtung des diffraktiven optischen Elements (104, 406) konfiguriert ist.

4. Optische Sendervorrichtung nach Anspruch 3, wobei die Projektorsonde die Gradientenindex- (GRIN) Linse (402) zwischen dem Lichtausgangsende der optischen Faser und dem DOE (406) umfasst.

5. Optische Sendervorrichtung nach einem von Anspruch 3 oder Anspruch 4, wobei die Lichtleitstruktur einen Strahlenausgang mit einem Strahlenausgangsfleckdurchmesser von mindestens etwa 4 $\mu$m aufweist und wobei die Lichtquelle mit dem Projektor (26', 46, 56, 66, 76, 106, 206, 406) betriebsmäßig so verbunden ist, dass ein Lichtstrahl auf den Projektor auftrifft, wobei der auftreffende Lichtstrahl einen Strahldurchmesser von mindestens etwa 0,4 mm, wie beispielsweise mindestens etwa 0,6 mm, wie beispielsweise mindestens etwa 0,7 mm aufweist.

6. Optische Sendervorrichtung nach einem der vorstehenden Ansprüche, wobei die optische Sendervorrichtung eine Spitzenschutzanordnung umfasst, die so konfiguriert ist, dass sie die Spitze (64) vollständig oder teilweise abdeckt, nachdem der distale Abschnitt (62) des Durchdringungselements die Haut durchdrungen hat und in eine Körperhöhle eingetreten ist, wobei die Spitzenschutzanordnung vorzugsweise einen Flansch (67a) umfasst, der optional einen Teil des Projektors und/oder der Projektorsonde bildet.

7. Optische Sendervorrichtung nach einem der vorstehenden Ansprüche, wobei die optische Sendervorrichtung eine Intra-Höhlen-Schutzanordnung zum Schutz vor unerwünschten Einwirkungen durch die Durchdringungsspitze in einer Körperhöhle umfasst, wobei die Intra-Höhlen-Schutzanordnung vorzugsweise so konfiguriert ist, dass sie die Durchdringungsspitze zwischen einer ersten Durchdringungsposition und einer zweiten Durchdringungsspitzen-Schutzposition umschaltet.

8. Optische Sendervorrichtung nach einem der vorstehenden Ansprüche, wobei der Projektor (26', 46, 56, 66, 76, 106, 206, 406) so konfiguriert ist, dass er das Lichtmuster mit einem Divergenzwinkel von mindestens etwa 10 Grad, wie beispielsweise mindestens etwa 20 Grad, wie beispielsweise mindestens etwa 30 Grad, wie beispielsweise mindestens etwa 40 Grad relativ zu der optischen Achse des Lichtmusters projiziert, wobei der Divergenzwinkel vorzugsweise wählbar ist.

9. Optische Sendervorrichtung nach einem der vorstehenden Ansprüche, wobei das Lichtmuster strukturiertes Licht umfasst, das Bereiche mit Licht und Bereiche ohne Licht und/oder Bereiche mit Licht einer ersten Qualität einer Eigenschaft und Bereiche mit Licht einer zweiten Qualität der Eigenschaft umfasst, wobei die Eigenschaft vorteilhafterweise aus Lichtintensität, Wellenlänge und/oder Wellenlängenbereich ausgewählt ist.

10. 3D-Sensorsystem, das eine optische Sendervorrichtung nach einem der vorstehenden Ansprüche und einen zugehörigen Detektor (99) umfasst, wobei der zugehörige Detektor (99) zum Detektieren von reflektierten Strahlen des von dem Projektor der optischen Sendervorrichtung projizierten Lichtmusters konfiguriert ist.

11. 3D-Sensorsystem nach Anspruch 10, wobei das 3D-Sensorsystem ein Computersystem (98) umfasst, wobei das Computersystem betriebsmäßig mit der optischen Sendervorrichtung (81, 91) und dem zugehörigen Detektor (99) verbunden ist und zum Berechnen von Daten konfiguriert ist, die eine 3D-Oberflächenkontur eines Oberflächenbereichs darstellen, der von der optischen Sendervorrichtung gesendetes Licht reflektiert, basierend auf 3D-Signaldaten von der optischen Sendervorrichtung und detektierten Daten von dem zugehörigen Detektor.

12. 3D-Sensorsystem nach Anspruch 11, wobei das Computersystem (98) so konfiguriert ist, dass es Winkelpositionsdaten empfängt, die den Winkel zwischen der optischen Achse des Projektors und der optischen Achse des zugehörigen Detektors (99) darstellen, und/oder das Computersystem so konfiguriert ist, dass es Daten zum Berechnen der Winkelpositionsdaten empfängt.

13. 3D-Sensorsystem nach einem der Ansprüche 10 - 12, wobei das 3D-Sensorsystem einen Teil eines Bedieners in Form eines Roboters bildet, wobei die Sendervorrichtung (81, 91) und der zugehörige Detektor mit dem Roboter verbunden sind oder in diesen integriert sind und das 3D-Sensorsystem für einen Echtzeitbetrieb konfiguriert ist, wobei das Computersystem für eine Echtzeitberechnung von Daten konfiguriert ist, die die 3D-Oberflächenkontur darstellen, die eine 3D-Rekonstruktion mindestens eines Teils einer Körperhöhle darstellt.

14. 3D-Sensorsystem nach einem der Ansprüche 11 - 13, wobei das Computersystem (98) so konfiguriert ist, dass es Daten vor dem Betrieb und/oder Intra-Betriebs-Daten empfängt, die mindestens ein Attribut der Körperhöhle darstellen, und dass es die Daten vor dem Betrieb zur Berechnung von Daten verwendet, die eine 3D-Rekonstruktion mindestens eines Teils einer Körperhöhle darstellen, wobei die Daten vor dem Betrieb vorzugsweise Bilddaten und/oder Abtastdaten und/oder entsprechende geschätzte Daten, berechnete Daten und/oder Abtastdaten umfassen, die während des minimalinvasiven chirurgischen und/oder diagnostischen Verfahrens erhalten werden.

**Revendications**

1. Dispositif émetteur optique destiné à émettre de la lumière à l'intérieur d'une cavité corporelle, ledit dispositif émetteur optique (81, 91) comprenant un élément pénétrateur allongé (1, 11, 31, 71) destiné à pénétrer à travers de la peau mammalienne et un élément éclairant structuré associé, l'élément pénétrateur(1, 11, 31, 71) présente une partie distale (2, 12, 22, 32, 42, 52, 62, 72, 82, 92) et une partie proximale (3, 33, 73, 83, 93), la partie distale (2, 12, 22, 32, 42, 52, 62, 72, 82, 92) ayant un axe central et une longueur (L) et comprenant une pointe (4, 14, 24, 34, 44, 54, 64, 74, 84) de pénétrateur, l'élément éclairant structuré comprend une source de lumière et un projecteur (26', 46, 56, 66, 76, 106, 206, 406), dans lequel la source de lumière est fonctionnellement reliée au projecteur (26', 46, 56, 66, 76, 106, 206, 406) par une structure de guidage de lumière (201, 301, 401), la structure de guidage de lumière et le projecteur font partie d'une sonde à projecteur, le projecteur (26', 46, 56, 66, 76, 106, 206, 406) est disposé à la partie distale (2, 12, 22, 32, 42, 52, 62, 72, 82, 92) de l'élément pénétrateur (1, 11, 31, 71) et est configuré pour projeter un motif lumineux divergent et dans lequel le projecteur comprend un élément optique diffractant (EOD) (104) comprenant une surface mi-crostructurée configurée pour créer le motif lumineux et comprend en outre un dispositif de lentille à expansion de faisceau, comprenant une lentille à gradient d'indice (GRIN) (402) disposée avant l'EOD (104, 406), de préférence le projecteur (26', 46, 56, 66, 76, 106, 206, 406) présente au moins une position dans laquelle il est configuré pour projeter le motif lumineux vers l'extérieur par rapport au dispositif émetteur optique, de préférence pour projeter le motif lumineux distalement par rapport au dispositif émetteur optique.

2. Dispositif émetteur optique selon la revendication 1, dans lequel ladite partie distale (2, 12, 22, 32, 42, 52, 62, 72, 82, 92) de l'élément pénétrateur a une longueur (L) d'au moins environ 0,5 cm et une dimension maximale de section transversale de jusqu'à environ 1 cm, telle que de jusqu'à environ 2 mm.

3. Dispositif émetteur optique selon l'une quelconque des revendications précédentes, dans lequel ladite structure de guidage de lumière comprend une fibre optique (201, 301, 401) présentant une extrémité de sortie de lumière configurée pour transmettre un faisceau de lumière de sortie vers l'élément optique diffractant (104, 406).

4. Dispositif émetteur optique selon la revendication 3, dans lequel ladite sonde à projecteur comprend ladite lentille à gradient d'indice (GRIN) (402) entre l'extrémité de sortie de lumière de fibre optique et ledit EOD (406).

5. Dispositif émetteur optique selon l'une quelconque des revendications 3 et 4, dans lequel ladite structure de guidage de lumière présente une sortie de faisceau ayant un diamètre de point de sortie de faisceau d'environ 4 μm et dans lequel ladite source de lumière est fonctionnellement reliée au projecteur (26', 46, 56, 66, 76, 106, 206, 406) de sorte qu'un faisceau de lumière est incident sur le projecteur, ledit faisceau de lumière incident ayant un diamètre de faisceau d'au moins environ 0,4 mm, tel que d'au moins environ 0,6 mm, tel que d'au moins environ 0,7 mm.

6. Dispositif émetteur optique selon l'une quelconque des revendications précédentes, ledit dispositif émetteur optique comprenant un dispositif protecteur de pointe configuré pour couvrir entièrement ou partiellement la pointe (64) après que la partie distale (62) de l'élément pénétrateur a pénétré à travers la peau et pénétré dans une cavité corporelle, ledit dispositif protecteur de pointe comprenant de préférence une bride (67a) faisant éventuellement partie du projecteur et/ou de la sonde à projecteur.

7. Dispositif émetteur optique selon l'une quelconque des revendications précédentes, ledit dispositif émetteur optique comprenant un dispositif protecteur intra-cavité destiné à protéger contre des impacts indésirables par la pointe de pénétrateur à l'intérieur d'une cavité corporelle, de préférence le dispositif protecteur intra-cavité étant configuré pour basculer la pointe de pénétrateur entre une première position de pénétrateur et une seconde position protégée de pointe de pénétrateur.

8. Dispositif émetteur optique selon l'une quelconque des revendications précédentes, dans lequel ledit projecteur (26', 46, 56, 66, 76, 106, 206, 406) est configuré pour projeter le motif lumineux avec un angle divergent d'au moins environ 10 degrés, tel que d'au moins environ 20 degrés, tel que d'au moins environ 30 degrés, tel que d'au moins environ 40 degrés, par rapport à l'axe optique du motif lumineux, de préférence l'angle divergent est sélectionnable.

9. Dispositif émetteur optique selon l'une quelconque des revendications précédentes, dans lequel ledit motif lumineux comprend des zones de lumière comprenant de la lumière structurée et des zones sans lumière et/ou des zones de lumière d'une première qualité d'une caractéristique et des zones de lumière d'une seconde qualité de la caractéristique, la caractéristique étant avantageusement choisie parmi l'intensité, la longueur d'onde et/ou la gamme de longueurs d'onde de lumière.

10. Système de capteur 3D comprenant un dispositif émetteur optique selon l'une quelconque des revendications précédentes et un détecteur associé (99), dans lequel ledit détecteur associé (99) est configuré pour détecter des rayons réfléchis du motif lumineux projeté par le projecteur du dispositif émetteur optique.

11. Système de capteur 3D selon la revendication 10, ledit système de capteur 3D comprenant un système informatique (98), ledit système informatique étant fonctionnellement relié audit dispositif émetteur optique (81, 91) et audit détecteur associé (99) et étant configuré pour calculer des données représentant un contour de surface 3D d'une zone de surface réfléchissant de la lumière émise par ledit dispositif émetteur optique sur la base de données de signaux 3D provenant dudit dispositif émetteur optique et de données détectées provenant dudit détecteur associé.

12. Système de capteur 3D selon la revendication 11, dans lequel ledit système informatique (98) est configuré pour recevoir des données de position angulaire représentant l'angle entre l'axe optique du projecteur et l'axe optique du détecteur associé (99) et/ou le système informatique est configuré pour recevoir des données afin de calculer lesdites données de position angulaire.

13. Système de capteur 3D selon l'une quelconque des revendications 10-12, dans lequel ledit système de capteur 3D fait partie d'un opérateur sous la forme d'un robot, ledit dispositif émetteur (81, 91) et ledit détecteur associé étant reliés audit ou étant intégré audit robot et ledit système de capteur 3D étant configuré pour fonctionnement en temps réel, ledit système informatique étant configuré pour calculer en temps réel des données représentant ledit contour de surface 3D représentant une reconstruction en 3D d'au moins une partie d'une cavité corporelle.

14. Système de capteur 3D selon l'une quelconque des revendications 11-13, dans lequel ledit système informatique (98) est configuré pour recevoir des données préopératoires et/ou des données peropératoires représentant au moins un attribut de la cavité corporelle et pour utiliser lesdites données préopératoires afin de calculer des données représentant une reconstruction en 3D d'au moins une partie d'une cavité corporelle, lesdites données préopératoires comprenant de préférence des données d'image et/ou des données de balayage et/ou des données estimées correspondantes, des données calculées et/ou des données de balayage obtenues pendant la chirurgie mini-invasive et/ou la procédure de diagnostic.

Fig. 1

Fig. 2

Fig. 2a  Fig. 2b  Fig. 2c  Fig. 2d

Fig. 3

Fig. 4a

EP 3 558 147 B1

Fig. 4b

EP 3 558 147 B1

Fig. 4c

Fig. 5

Fig. 6a

Fig. 6b

Fig. 6c

Fig. 7

Fig. 8a

Fig. 8b

Fig. 8c

Fig. 9a

Fig. 9b

Fig. 10a

Fig. 10b

Fig. 10c

Fig. 10d

Fig. 10e

Fig. 11

91  93
00
92
P
99
P1

image output
via camera

Calibration to
camera

Algorithms for 3D
data set (point
cloud)

topography data
in real-time

Pre operation
data intra operation
data

PC

98

3D reconstruction, augmented
reality, volumetric measures,
instrument tracking, etc.

Fig. 12

Fig. 13a

Fig. 13b

Fig. 13c

Fig. 13d

Fig. 13e

Fig. 13f

Fig. 14a

Fig. 14b

Fig. 15

301 302 r2

r1

Fig. 16

Fig. 17

EP 3 558 147 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20130296712 A
- WO 2013163391 A
- US 2008071140 A
- US 2010268067 A
- US 2011069159 A
- US 20110110114 A
- EP 3056934 A1
- PA 201770631
- WO 2015124159 A

**Non-patent literature cited in the description**

- **STÉPHANE NICOLAU et al.** Augmented reality in laparoscopic surgical oncology. *Surgical Oncology*, 2011, vol. 20, 189-201
- **CHOI HYUNSEOK et al.** An effective visualization technique for depth perception in augmented reality-based surgical navigation. *The international journal of medical robotics and computer assisted surgery*, 05 May 2015
- **C. ROCCHIHI et al.** A low cost 3D scanner based on structured light. *Eurographics*, 2001, vol. 20 (3)